(19) Europäisches Patentamt
European Patent Office
Office européen des brevets

(11) **EP 4 162 928 A1**

(12) **EUROPEAN PATENT APPLICATION**

(43) Date of publication:
**12.04.2023 Bulletin 2023/15**

(21) Application number: **21203010.0**

(22) Date of filing: **15.10.2021**

(51) International Patent Classification (IPC):
*A61K 9/08* (2006.01)   *A61P 1/00* (2006.01)
*A61K 47/10* (2006.01)   *A61K 47/36* (2006.01)
*A61K 47/38* (2006.01)

(52) Cooperative Patent Classification (CPC):
**A61K 9/0031; A61K 9/08; A61K 31/395;
A61K 47/10; A61K 47/36; A61K 47/38; A61P 1/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.10.2021 US 202163252315 P**

(71) Applicant: **Cosmo Technologies Ltd.
Dublin 2 (IE)**

(72) Inventors:
• **GERLONI, Mara**
  **San Diego (US)**
• **MACELLONI, Cristina**
  **Bovisio Masciago (IT)**
• **ROSETTE, Caridad**
  **San Diego (US)**

(74) Representative: **FRKelly**
  **27 Clyde Road**
  **Dublin D04 F838 (IE)**

(54) **RIFAMYCIN FOR THE TREATMENT OF POUCHITIS, WITH AN IN-SITU GELLING FORMULATION**

(57)    The present application relates to an in situ enema containing Rifamycin SV and its use in the treatment of pouchitis. More particularly, the present application describes an in situ gelling retentive enema containing Rifamycin SV for treating, ameliorating, reducing the severity of or slowing the progression of pouchitis.

Fig. 17 stimulation of PXR transcriptional activity in reporter cell line by Rifamycin gel and Rifamycin SV

Processed by Luminess, 75001 PARIS (FR)

**Description**

**BACKGROUND**

The present application relates to an in-situ gelling enema containing Rifamycin SV (also referred to as rifamycin herein) and its use in the treatment of pouchitis, proctitis and distal ulcerative colitis (UC). More particularly, the present application describes a long-lasting in situ gelling retentive enema containing Rifamycin SV for treating, ameliorating, reducing the severity of or slowing the progression of pouchitis.

The publications and other materials used herein to illuminate the background or provide additional details respecting the practice, are incorporated by reference, and for convenience are respectively grouped in the Bibliography.

A potentially curative surgical option for both ulcerative colitis (UC) and familial adenomatous polyposis (FAP) is a restorative proctocolectomy (RPC) preserving the anal sphincters, followed by an ileal pouch-anal anastomosis (IPAA), which recreates a faecal reservoir [Li and Shen, 2014]. Although this surgery has improved patient quality of life and significantly reduced the risk of dysplasia or neoplasia in ulcerative colitis patients, complications are common.

Pouchitis is an inflammatory disorder of the ileal pouch, the artificial rectum surgically created out of ileal gut tissue. It is the most common long-term complication of ileal pouch surgery and has a significant adverse impact on patient quality of life [Li and Shen, 2014]. About 50% patients with an IPAA for UC develop at least one episode of subsequent inflammation of the ileal pouch (pouchitis) that may evolve to a long-term complication [Li and Shen, 2014]. Contrarily, just 0-11% of FAP patients develop pouchitis. This data provides evidence that pouchitis is less related to the structure of the pouch, but is a function of the patients' underlying immune dysregulation interacting with the pouch

Pouchitis, similar to inflammatory bowel disease, is a complex disorder that is not caused by any one single factor. More likely, pouchitis occurs through a combination of both dysregulated host inflammatory mechanisms and interaction with luminal microbiota. Bacterial overgrowth and faecal stasis in the small intestine may contribute to the development of pouchitis. Thus, most patients with pouchitis are treated with antibiotics.

The most frequently indicative symptoms of pouchitis are dysfunctions associated with increased frequency of evacuations, reduction in fecal consistency, rectorrhagia, pain, cramp-like abdominals, urgency, tenesmus, fecal incontinence, fever and extraintestinal manifestations. A clinical diagnosis should ideally be confirmed by endoscopy and mucosal biopsy of the pouch. Endoscopic examination shows inflammatory changes that may include mucosal edema, granularity, contact bleeding, loss of vascular pattern, hemorrhage, and ulceration. Histologic examination shows acute inflammation, including neutrophil infiltration and mucosal ulceration superimposed on a background of chronic inflammation including villous atrophy, crypt hyperplasia, and chronic inflammatory cell infiltration.

Pouchitis is classified as acute or chronic pouchitis. Acute pouchitis is defined as symptoms lasting less than 4 weeks (≤4 weeks) and responding to 2-week courses of antibiotics. Chronic pouchitis is defined as having symptoms lasting longer than four weeks (>4 weeks) despite standard antibiotic courses and requiring chronic antibiotics or anti-inflammatory therapy [Isaacs, et al., 2007]. Approximately 10% to 15% of patients with acute pouchitis develop chronic pouchitis which has subgroups such as antibiotic-dependent pouchitis and antibiotic-refractory pouchitis.

The diagnosis of pouchitis is based on the combined assessment of symptoms, endoscopic, and histologic findings. Sandborn et al. (1994) proposed the pouchitis disease activity index (PDAI), which is a 19-point index of pouchitis activity based on both clinical symptoms and endoscopic and histologic findings. Active pouchitis is defined as a PDAI ≥7, and remission is defined as a PDAI <7 [Nguyen, 2019]. A later study suggested that the omission of histological scores from PDAI (modified PDAI) can offer similar diagnostic accuracy when compared with the PDAI for patients with acute pouchitis [Akiyama et al., 2021].

The pathogenesis of pouchitis is not definitively understood, but various hypotheses have been proposed, including: (1) recurrence of UC, (2) dysbiosis of the ileal pouch microbiota, (3) deprivation of nutritional short chain fatty acids, (4) mucosal ischemia and oxygen free radical injury, (5) host genetic susceptibility, and (6) immune dysregulation. However, none of these alone can fully explain pouchitis pathogenesis. It has been suggested that pouchitis may represent a novel third form of inflammatory bowel disease (IBD).Similar to IBD, pouchitis is a complex disorder that is not caused by any one single factor. More likely, pouchitis occurs through a combination of both dysregulated host inflammatory mechanisms and interaction with luminal microbiota. Bacteria have been implicated in the disease process as bacterial overgrowth and faecal stasis in the small intestine may contribute to the development of pouchitis.

Although pouchitis is not well understood, most patients with pouchitis are treated with antibiotics. Although antibiotics are of some clinical benefit in Crohn's disease (CD), they are much less so than in pouchitis where they are more effective than any other therapy. While many different treatments for pouchitis have been reported with varying results, antibiotic treatment remains the most studied and is the mainstay of treatment.

Pouchitis is classified into three categories based on the response to standard antibiotic-based medical therapy: (1) antibiotic-responsive, (2) antibiotic-dependent, and (3) antibiotic-refractory. Patients who respond favorably to a 10-14 day course of antibiotics are considered to be antibiotic responsive. About 7-19% of pouchitis patients will require long-term continuous use of antibiotics to maintain pouchitis remission and these individuals are considered antibiotic-dependent. A small percentage of patients (<5%) may be classified as antibiotic-refractory. These patients do not respond to antibiotic therapy and in many cases, require aminosalicylates (e.g. mesalamine), immunosuppressive (e.g. 6- mercaptopurine), or biologics to induce or maintain remission [Schieffer, et al., 2016].

The treatment of pouchitis is mainly oral, but several products are in development for local treatment, such as enemas and gels, including in situ gels. For example, CN102151242 describes an in-situ gel sustained-release preparation comprising microspheres of active ingredient and a temperature-ion-sensitive type in situ-gel. The active ingredients identified include Rifampicin in the lists off active ingredients.

Al-Joufi, F., et al. (2021) discloses rectal mucoadhesive and in situ rectal gels containing, e.g., Rifampicin.

WO 2019/122253 discloses a liquid composition that can be a structured viscous composition or a soft gel containing two or three defined polymers for in situ delivery or release of an active agent. The active agent may be antimicrobics or antibiotics selected from, among others, Rifamycin SV, Rifaximin, and Rifampicin. The liquid composition may be used in the diagnosis, prevention, alleviation, treatment and/or reduction of pathologies or disorders affecting the human body including those affecting the gastro-intestinal tract, such as disorders that are inflammatory and/or degenerative pathologies. Example 11 describes a liquid composition containing three defined polymers and rifamycin SV for treating fistulas.

Rifamycin SV is a semisynthetic antibacterial belonging to the ansamycin class; it exerts its antibacterial activity by irreversibly binding to the bacterial DNA-dependent RNA polymerase $\beta$-subunit, thereby inhibiting bacterial RNA synthesis. Rifamycin SV exhibits an antibacterial activity against most bacterial enteropathogens frequently associated with infectious diarrhoea and other enteric infections, including Enterobacteriaceae and non-Enterobacteriaceae [Clin Drug Investig 2019 Jul., 39(7):691-697]. Rifamycin SV corresponds to the sodium salt of Rifamycin or Rifamycin sodium. For colonic diseases like diverticulitis, inflammatory bowel syndrome (IBS) or inflammatory bowel disease (IBD), bacterial proliferation or microbial dysbiosis is associated with a strong inflammatory component. This inflammation has a profound influence on the liver via the gut-liver axis. Rifamycin is endowed with anti-inflammatory activities based on the impact on two key regulators of inflammation: pregnane X receptor (PXR) and nuclear factor-KB (NF$\kappa$B). Rifamycin was found to activate PXR and two of its downstream targets, cytochrome P450 3A4 (CYP3A4) and P-glycoprotein (PgP), in liver and intestinal cell lines. Rifamycin also directly inhibited NF$\kappa$B in a cell line which lacks PXR expression [Rosette, et al., 2019]. These dual activities likely explain the inhibition of pro-inflammatory cytokine secretion from human colonic cells lines and activated CD4+ T cells [Rosette, et al., 2013]. Rifamycin exerts its clinical benefit as topical action into the intestinal tract (J-Pouch, or S-pouch, or W-pouch in case of pouchitis) and it is characterized by a negligible absorption in the bloodstream. Rifamycin is a safe drug and has a favorable safety profile as compared to for example ciprofloxacin and metronidazole currently used in the treatment of gastrointestinal diseases with an inflammatory component, such as pouchitis, or proctitis, or ulcerative colitis.

Proctitis and/or distal ulcerative colitis are inflammatory diseases frequently associated to IBD in terms of symptoms and causes. Proctitis is the inflammation of the lining of the rectum. Depending on the cause, proctitis may happen suddenly and last a short time or may be long lasting with frequent relapsing episodes. As per IBD in general, bacterial overgrowth and/or dysregulation may be a worsening factor. Distal Ulcerative Colitis defines diseases distal to the splenic flexure which include proctitis (involvement of the rectum only), proctosigmoiditis (involvement of rectum and sigmoid colon) and left-sided colitis (involvement extending as far as descending colon or splenic flexure).

SUMMARY

The present application relates to an in-situ gelling enema containing Rifamycin SV (also referred to as rifamycin herein) and its use in the treatment of pouchitis and/or proctitis and/or distal ulcerative colitis. More particularly, the present application describes a long-lasting in situ gelling enema containing Rifamycin SV for treating, ameliorating, reducing the severity of, inducing, maintaining the remission of, and/or slowing the progression of pouchitis.

In one aspect, the invention relates to a pharmaceutical composition comprising an active agent and a polymeric mixture as described herein. The pharmaceutical composition is able to gel in situ upon contact with the target site of action (distal part of the gastro-intestinal tract and/or the artificial cavity surgically created - e.g., ileal pouch) ensuring the prolongation of the contact time with the mucosa therein ensuring a sustained release of the active agent topically over time. This means that the pharmaceutical composition is able to provide a composition that becomes, upon application on the intestinal mucosa, a long-lasting gel acting over time at the targeted site. In one embodiment, the polymeric mixture comprises at least one thermo-responsive polymer (first polymer), at least one ion-sensitive polymer (second polymer) and at least one bio-adhesive polymer (third polymer).

In one embodiment, the first polymer is selected from the group comprising but not limited to: polyoxyethylene-polyoxypropylene block copolymers, such as poloxamer 124, poloxamer 188, poloxamer 237, poloxamer 338, poloxamer 407 and the like, poly(ethylene glycol)/poly(lactide-coglicolide) block copolymers (PEG-PLGA), poly(ethylene glycol)-poly(lactic acid)-poly(ethylene glycol) (PEG-PLA-PEG), poly(N-isopropylacrylamide), and cellulose derivatives, like methylcellulose (MC) and hydroxypropylmethylcellulose (HPMC) and the like and mixtures thereof.

In another embodiment, the second polymer is selected from the group comprising but not limited to: carrageenan, gellan gum, pectin, alginate, alginate salts and the like, and mixtures thereof.

In a further embodiment, the third polymer is selected from the group comprising, but not limited to: chitosan, hyaluronic acid and salts thereof, cellulose derivatives, such as methyl cellulose, hydroxy propyl methylcellulose, hydroxy propyl cellulose, carboxymethyl cellulose sodium and the like, polyvinyl alcohol, polyvinyl pyrrolidone, polyacrylic acid, polyethylene oxides, cyclodextrins, tragacanth, sodium alginate, xanthan gum, gelatin, pectin, and the like and mixtures thereof.

The pharmaceutical composition may further comprise at least one other excipient such as for example antioxidants, chelating agents, preservatives, antimicrobial agents, surfactants, co-surfactants, lipophilic compounds, purified water, organic salts, inorganic salts, buffers agents or mixtures thereof. In a preferred embodiment, the pharmaceutical composition further comprises at least one antioxidant and a least one preservative.

In a second aspect, the invention relates to the use of the pharmaceutical composition as a long-lasting in situ gelling enema for treating, ameliorating, reducing the severity of, slowing the progression of, inducing the remission of, or maintaining the remission of pouchitis. In a preferred embodiment, the active agent is Rifamycin SV.

In one embodiment, the long-lasting in situ gelling enema is a retentive enema able to provide a sustained release of the active ingredient ensuring that the drug substance remains embedded in the thin layer of the gel in tight contact with the site of action, prolonging the duration of its pharmacological activity in situ.

## BRIEF DESCRIPTION OF THE FIGURES

The drawings directly relate to the Examples set out herein and are explained in detail later in this document.

Fig. 1 shows the factor space of a DoE approach, "simplex centroid design", used to identify the optimal quantitative composition of the polymer mixture.

Fig. 2 shows viscosity curves of Runs from 1 to 6 and Runs from 8 to 9 with Rifamycin SV both at 25 °C and 37 °C.

Figs. 3 shows the temperature ramp for Run 2 at 10 Hz.

Fig. 4 shows Fmax values for Runs 1-10 in presence and absence (blank) of mucine at 8% w/w.

Fig. 5 shows Work of Adhesion for Runs 1-10 in presence and absence (blank) of mucine at 8% w/w.

Fig. 6 shows contour plot of normalized Work of Adhesion.

Fig. 7 shows the factor space of the DoE approach, "full factorial design" used to identify the optimal quantitative composition of the polymer mixture.

Fig. 8 shows logarithmic viscosity curve of Runs 1-14 of "full factorial design" with Rifamycin SV.

Fig. 9 shows the temperature ramp of Vehicle 9.

Fig. 10 shows the temperature ramp of Vehicle 14.

Fig. 11 shows contour plot of normalized Work of Adhesion.

Fig. 12 shows loss tangent (Tan $\delta$) as a function of frequency at 25 °C and 40 °C.

Fig. 13 shows time dependency profiles of both Placebo and Rifamycin SV formulations.

Fig. 14 shows a viscosity curve at 37°C comparing the mixture of polymers without Rifamycin SV (vehicle) with the pharmaceutical compositions comprising 0.5% and 1% Rifamycin SV.

Fig. 15 shows Rifamycin SV MIC values tested on bacteria involved in pouchitis.

Fig. 16 shows bacterial growth inhibition halos (mm) for Rifamycin Gels and MIC ($\mu$g/mL) for Rifamycin SV.

Fig. 17 shows stimulation of PXR transcriptional activity in reporter cell line by Rifamycin Gel and Rifamycin SV.

Fig. 18 shows inhibition of mTOR and S6R phosphorylation in human primary colonocytes.

## DETAILED DESCRIPTION

The present application relates to a formulation containing Rifamycin SV having a therapeutic application and preferably to an in-situ gelling enema containing Rifamycin SV (also referred to as Rifamycin or Rifamycin sodium) and its use in the treatment of diseases affecting the distal part of the gastrointestinal tract. More particularly, the present application describes a long-lasting in situ gelling enema containing Rifamycin SV and its use for treating, ameliorating, reducing the severity of or slowing the progression of, inducing and/or maintaining the remission of gastrointestinal diseases of the distal part of the gastrointestinal tract. In some embodiments, the long-lasting in situ gelling enema is a retentive enema. In some embodiments, the long-lasting in situ gelling enema provides a sustained and/or prolonged action of the active ingredient at the site of action.

In some embodiments, such distal part of the gastrointestinal tract comprises the distal colon and rectum, more specifically the sigmoid colon and rectum. According to such embodiments, such diseases affecting the distal part of the gastrointestinal tract, more specifically the sigmoid colon and rectum, can be selected from the group comprising proctitis and/or distal ulcerative colitis. In another embodiment, such distal part of the gastrointestinal tract comprises an artificial cavity created surgically, from disease or resulting from injury. In one embodiment, the cavity is an ileal pouch (J-pouch or S-Pouch or W-Pouch) created by ileoanal anastomosis (J-Pouch or S-Pouch or W-Pouch) surgery or ileostomy surgery (K-Pouch). According to such embodiment, the disease affecting the distal part of the gastrointestinal tract, more specifically the ileal pouch, is pouchitis.

In one embodiment, the present application describes a long-lasting in situ gelling enema containing Rifamycin SV for treating, ameliorating, reducing the severity of or slowing the progression of pouchitis.

In one embodiment, the present application describes a long-lasting in situ gelling enema containing Rifamycin SV for treating, ameliorating, reducing the severity of, inducing and/or maintaining the remission of, or slowing the progression of proctitis. In another embodiment, the present application describes a long-lasting in situ gelling retentive enema containing Rifamycin SV for treating, ameliorating, reducing the severity of, inducing and/or maintaining the remission of, or slowing the progression of distal ulcerative colitis. In a preferred embodiment, the present application describes a long-lasting in situ gelling retentive enema containing Rifamycin SV for treating, ameliorating, reducing the severity of, inducing and/or maintaining the remission of, or slowing the progression of pouchitis.

In one aspect, the invention relates to a pharmaceutical composition comprising Rifamycin SV and a polymer mixture as described herein. The pharmaceutical composition is able to gel in situ upon contact with the target site ensuring the sustained and/or prolonged release of the active agent topically over time at the targeted site. In one embodiment, the polymer mixture comprises at least one thermo-responsive polymer (first polymer), at least one ion-sensitive polymer (second polymer) and at least one bio-adhesive polymer (third polymer).

The mechanisms of action of these polymers are different. The thermo-responsive polymer (first polymer) gels or is able to create a structure into the body or body cavity when the temperature reaches a suitable range of values. The ion-sensitive polymer (second polymer) gels or is able to create a structure with the bio-relevant medium in the presence of specific ions in suitable concentrations. The bio-adhesive polymer (third polymer) provides improved adhesion of the pharmaceutical composition and improved residence time at the target site. The bio-adhesive polymer (third polymer) ensures the improved adhesion through its interaction with the mucosa of the distal part of the gastrointestinal tract and/or the artificial cavity surgically created (ileal pouch). Together, the bio-adhesive polymer enhances the grafting of the pharmaceutical composition to the body tissue, whereas the first and second polymers enhance the structure of the gelling thin layer obtained by spreading the pharmaceutical composition on the target body surface to act as a reservoir or a tridimensional matrix system to ensure the persistence and the prolonged pharmacological activity.

In one embodiment, the first polymer gels or creates a structured state after contact with the bio-relevant medium or at physiological temperature (i.e., at about 37 °C); the action of the first polymer is further strengthened by the interaction of the second polymer with the target site, where the presence of specific trigger ions strengthens the interaction with the environmental medium of the other, resulting in an expansion of the polymer mixture's capability of forming a gel or a structured state. Upon contact with a site into the body or body cavity (for example, distal part of the gastro-intestinal tract and/or the artificial cavity surgically created - e.g., ileal pouch), characterized by a physiological temperature (i.e., $\geq 34°C$), the third polymer further ensures and strengthens the bio-adhesion grafting the pharmaceutical composition to the body tissue. The polymer mixture is effective because of an optimal mixture of the first, second and third polymers that lead to a synergism between the polymers themselves and the polymers with the microenvironment, leading to a strengthened gelation/structuring of the pharmaceutical composition at the targeted site. Such strengthened gelation is demonstrated by a huge increase in the viscoelastic modulus G', as described in WO 2019/122593, incorporated herein by reference in its entirety.

It has been discovered that Rifamycin SV actively interacts with the polymer mixture and this interaction has been investigated by using a Mixture Design model as set forth in the Examples. The evaluation of rheological properties for an in-situ gelling formulation is one of the most important parameters able to predict the behaviour in vivo. The rheological properties especially affect the ease of application and retention time in the targeted area. The rheological properties are determined at room temperature (25 °C) and physiological temperature (37 °C) to observe the changes in the gel structure. The structural and dynamical properties are investigated with the determination of G', storage modulus (measure of the elasticity) and G", loss modulus (representing viscous component).

Viscoelastic behaviors marked by a switch tendency between G' (storage modulus) and G" (viscous modulus) meaning a thermo gelation of the sample and measured through G' modulus (storage modulus) at 37°C is compared to G" (viscous modulus) at the same temperature. G' dominated G" at 37 °C and the gap between the two moduli is wider at 37 °C indicating the formation of a strong gel or structured state and an increment in terms of elasticity and confirming the transition from a liquid form at the application to a gel state or to a structured state post-application. The sol-gel transition allows the spreading and deposition of the formulation at the target site as a gel layer, with a subsequent enhanced adhesion property for a protracted suitable period of time. The sol-gel transition is mainly triggered by raising of the body temperature upon administration, preferably the lumen of the pouch in case of pouchitis, and by the interaction with the intestinal environment (mainly colonic fluid and/or the colon-like mucosa and/or the pouch) allowing the Rifamycin SV to remain embedded in the thin layer of the gel in tight contact with the site of action, and, prolonging the duration of its pharmacological activity in situ.

As described herein, the pharmaceutical composition comprises a polymer mixture comprising at least one thermo-responsive polymer (first polymer), at least one ion sensitive polymer (second polymer) and at least one bio-adhesive polymer (third polymer), so that the formulation can be easily administered to a patient in need thereof, while ensuring a prolonged permanence at the site of action for a desired period of time.

In the preparation of the pharmaceutical composition described herein, the choice of the suitable thermo-responsive polymers (first polymers) and of their concentration is made to obtain a final composition which is in liquid state below the body temperature (i.e. below about 37 °C, preferably at about 20 °C to about 25 °C) and which becomes a gel, or a structured state once exposed to the body temperature or above (i.e., at or above about 37 °C). Rifamycin SV, being an ionic large molecule, is known to potentially interact with biopolymers, altering the respective properties (e.g., thermogelling, and/or bioadhesive and/or ionotropic properties). The compositions of the invention, comprising a combination of the aforementioned three polymers, surprisingly allow to overcome this issue and to modulate the permanence time of said compositions at the target site.

In the preparation of the pharmaceutical composition described herein, the choice of the suitable ion-sensitive polymer (second polymer) and of their concentration is made to obtain a suitable sol-gel transition or a transition to a structured composition in the presence of specific ions.

As described herein, an ion-sensitive polymer can be selected among those able to bind mono and/or divalent inorganic ions, such as $Na^+$, $K^+$, $Mg^{2+}$, $Ca^{2+}$ and $Zn^{2+}$, and organic ions with high affinity. The responsiveness to ionic strength is a typical property of polymers containing ionizable groups. Changes in ionic strength may cause changes in the size of the polymeric micelles and polymer solubility.

When administered, the pharmaceutical composition according to the invention comprising an active agent, preferably Rifamycin SV, and a polymer mixture as described herein, is automatically heated to body temperature (i.e., about 37 °C). This increase in temperature triggers the transition of the pharmaceutical composition from a liquid to a gel state (sol-gel transition) or to a structured state due to the thermo-responsive first polymer; then, through the interaction of the ion sensitive second polymer with the ions present in the biorelevant medium and/or environment, said gel or structured state is further strengthened, as demonstrated by an increase in the viscoelastic modulus G'.

The transition from a liquid form to a gel state or a structured state enhances the bio-adhesiveness of the pharmaceutical composition, that is driven by the presence of said at least one bioadhesive polymer (third polymer) in the polymer mixture, with a subsequent enhancement of adhesion to the tissues, such as the intestinal or rectal mucosa for a suitable period of time as demonstrated by the muco-adhesion results at 37°C.

In addition, the increase of the viscoelastic modulus of the pharmaceutical composition slows down the diffusion of the at least one active agent, delaying its delivery to the affected site, with prolongation of the therapeutic effect.

When administered, the pharmaceutical compositions described herein are able to form a thin gel or structured layer (state) over the affected area, having a reduced tendency to flow along the organ walls. This property maximizes the contact time between the pharmaceutical compositions described herein, and the affected area: as a result, the active agent contained therein can remain in contact with the cell membrane of the epithelial cells of the mucosa for a longer period of time, in comparison with other known compositions.

In some embodiments, the pharmaceutical composition of the present invention may be used for the treatment of diseases of the distal part of the gastrointestinal tract, preferably where the target site is covered by a mucosal tissue allowing the grafting process. According to some embodiments, such distal part of the gastrointestinal tract comprises the distal colon and rectum, more specifically the sigmoid colon and rectum. According to such embodiments, such diseases affecting the distal part of the gastrointestinal tract, more specifically the sigmoid colon and rectum, can be selected from the group comprising: proctitis and/or distal ulcerative colitis. In another embodiment, such distal part of the gastrointestinal tract comprises an artificial cavity created surgically, from disease or resulting from injury. In one embodiment, the cavity is an ileal pouch (J-pouch or S-Pouch or W-Pouch) created by ileoanal anastomosis (J-Pouch or S-Pouch or W-Pouch) surgery or ileostomy surgery (K-Pouch). According to such embodiment, the disease affecting the distal part of the gastrointestinal tract, more specifically the ileal pouch, is pouchitis.

In an embodiment, the administration of the pharmaceutical composition of the present invention to a subject in need thereof induces the remission of proctitis. In another embodiment, the administration of the pharmaceutical compositions of the present invention to a subject in need thereof maintains the remission of proctitis. In another embodiment, the administration of the pharmaceutical compositions of the present invention to a subject in need thereof induces the remission of distal ulcerative colitis. In another embodiment, the administration of the pharmaceutical compositions of the present invention to a subject in need thereof maintains the remission of distal ulcerative colitis. In a preferred embodiment, the administration of the pharmaceutical compositions of the present invention to a subject in need thereof induces the remission of pouchitis. In another preferred embodiment, the administration of the pharmaceutical compositions of the present invention to a subject in need thereof maintains the remission of pouchitis.

The goal of pouchitis therapy is to alleviate symptoms and achieve and/or maintain the clinical and endoscopic remission by demonstrating mucosal healing. Histologic improvement (i.e., resolution of acute inflammatory cell infiltrate) is emerging as an additional component of disease remission. Endoscopic healing is an important goal of therapy because symptoms alone may not reflect the inflammatory status of the pouch. In an embodiment, the administration of the pharmaceutical compositions of the present invention to a subject in need thereof induces the remission of pouchitis. In another embodiment, the administration of the pharmaceutical compositions of the present invention to a subject in need thereof maintains the remission of pouchitis. For the purpose of the present invention, the induction of remission and the maintenance of remission must be intended as comprising, but not limited to, the alleviation or the amelioration or the reduction of the clinical symptoms, endoscopic symptoms or the histologic symptoms or a combination thereof. In one embodiment, the administration of the pharmaceutical composition alleviates and/or reduces and/or ameliorates one or more clinical symptoms selected from a group comprising, but not limited to: increased frequency of evacuations (also referred to as change in bowel habit), change in fecal consistency, rectorrhagia, hematochezia, pain, cramp-like abdominals, urgency, tenesmus, fecal incontinence, fever and/or extraintestinal manifestations, or combination thereof. In one embodiment, the administration of the pharmaceutical composition alleviates and/or reduces and/or ameliorates the occurrence of one or more endoscopy symptoms selected from the group comprising, but not limited to: mucosal edema, granularity, contact bleeding, loss of vascular pattern, hemorrhage, and ulceration. In an embodiment, the administration of the pharmaceutical composition alleviates and/or reduces and/or ameliorates the occurrence of one or more histologic symptoms selected from the group comprising, but not limited to: polymorph infiltration, ulceration, erosions, mononuclear leukocytes infiltration, and villous atrophy.

The treatment regimen and administration schedule of the composition of the present invention to subjects in need thereof may be adapted, depending upon the nature of the disease (e.g., acute or chronic), its severity and the overall condition of the patient. In one embodiment, the treatment period with the composition of the present invention may last for about 2 weeks to about 4 weeks for each cycle of treatment. In some embodiments, the treatment period with the composition of the present invention may last for about 1 week, 2 weeks, 3 weeks, or 4 weeks. In a preferred embodiment, the treatment period with the composition of the present invention may last 2 weeks. In another preferred embodiment, the treatment period with the composition of the present invention may last 4 weeks.

In some embodiments, the pharmaceutical composition of the present invention may be administered once daily, or twice a day, or three times a day, or four times a day. In a preferred embodiment, the pharmaceutical composition of the present invention is administered once daily. In another preferred embodiment, the pharmaceutical composition of the present invention is administered twice a day.

In one embodiment, the pharmaceutical composition of the present invention comprising 0.5% by weight of Rifamycin SV may be administered once daily, in a single dose 60 mL enema.

In one embodiment, the pharmaceutical composition of the present invention comprising 1.0 % by weight of Rifamycin SV may be administered once daily, in a single dose 60 mL enema.

In one embodiment, the pharmaceutical composition of the present invention comprising 0.5% by weight of Rifamycin SV may be administered twice a day, in a single dose 60 mL enema.

In one embodiment, the pharmaceutical composition of the present invention comprising 1.0% by weight of Rifamycin SV may be administered twice a day, in a single dose 60 mL enema.

In some embodiments, the treatment with the pharmaceutical composition of the present invention can be done once, to achieve the induction of remission, or maintain the remission of the disease in a subject in need thereof. In other embodiments, the treatment with the pharmaceutical compositions of the present invention can be repeated cyclically, to maintain the remission of the disease, and/or prevent the relapse of the disease, in a subject in need thereof. According to the latter embodiments, such cyclic treatments with the pharmaceutical composition of the present invention can be repeated once per month or every two months, or every three months, or every four months, or every five months, or every six months. According to a preferred embodiment, such cyclic treatments with the pharmaceutical composition of the present invention is repeated once per month. According to a preferred embodiment, such cyclic treatments with the pharmaceutical composition of the present invention is repeated once every two months. According to a further preferred embodiment, such cyclic treatments with the pharmaceutical composition of the present invention is repeated once every three months. The frequency of the cycles of treatments with the pharmaceutical composition of the present invention depend upon the frequency of relapse of the disease. The frequency of the cycles of treatments with the pharmaceutical composition of the present invention depend upon the maintenance of the remission. In some embodiments, the frequency of the treatments in case of maintenance of the remission is each month from two weeks to four weeks. The schedule of the administrations will be dependent on whether the pouchitis is acute or chronic, whether the patient is antibiotic-responsive or antibiotic-dependent.

In one embodiment, the at least one thermo-responsive polymer (first polymer) is selected from the group comprising but not limited to polyoxyethylene-polyoxypropylene block copolymers, such as poloxamer 124, poloxamer 188, poloxamer 237, poloxamer 338, poloxamer 407 and the like, poly(ethylene glycol)/poly(lactide-coglicolide) block copolymers (PEG-PLGA), poly(ethylene glycol)-poly(lactic acid)-poly(ethylene glycol) (PEG-PLA-PEG), poly(N-isopropylacrylamide), and cellulose derivatives, like methylcellulose (MC) and hydroxypropylmethylcellulose (HPMC) and the like, and mixtures thereof.

In a preferred embodiment, the at least one thermo-responsive polymer of the polymer mixture is poloxamer 188. In another preferred embodiment, the thermo-responsive polymer of the polymer mixture is poloxamer 407. In a further preferred embodiment, the thermo-responsive polymer of the polymer mixture comprises some mixtures of poloxamer 188 and poloxamer 407. In another preferred embodiment, the thermo-responsive polymer of the polymer mixture is a cellulose derivative, preferably methyl cellulose.

The amount of the at least one thermo-responsive polymer ranges between about 0.5% to about 30% by weight with respect to the weight of the liquid pharmaceutical composition, preferably between about 1% to about 25% by weight with respect to the weight of the liquid pharmaceutical composition, more preferably between about 12% and about 18% by weight with respect to the weight of the liquid pharmaceutical composition,

According to a preferred embodiment, said at least one thermo-responsive polymer is contained in an amount of about 0.5% or about 1.0% or about 5.0% or about 10.0% or about 14% or about 15% or about 16% or about 16.5% by weight with respect to the weight of the liquid pharmaceutical composition.

In one embodiment, the at least one ion sensitive polymer (second polymer) may be selected from the group of polysaccharides, comprising, but not limited to carrageenan, gellan gum, pectin, alginic acid and/or salts thereof. Any mixtures of these ion sensitive polymers can be used to form the appropriate polymer mixture. In a preferred embodiment, said at least one ion sensitive is alginic acid and/or salts thereof. In another preferred embodiment, said at least one ion sensitive polymer is sodium alginate.

In one embodiment, the at least one ion-sensitive polymer is contained in an amount which ranges from about 0.005 % to about 5% by weight with respect to the weight of the liquid pharmaceutical composition, more preferably from about 0.01% to about 3% by weight with respect to the weight of the liquid pharmaceutical composition, still more preferably from about 0.1% to about 2.0% by weight with respect to the weight of the liquid pharmaceutical composition.

In a preferred embodiment, the at least one ion-sensitive polymer is contained in an amount of about 0.1% or about 0.2% or about 0.3%, or about 0.4% or about 0.5% or about 1% or about 2% by weight with respect to the weight of the liquid pharmaceutical composition.

In one embodiment, the at least one bio-adhesive polymer (third polymer) is selected from the group comprising, but not limited to: chitosan, hyaluronic acid and salts thereof, cellulose derivatives, such methyl cellulose, hydroxy propyl methylcellulose, hydroxy propyl cellulose, carboxymethyl cellulose sodium and the like, polyvinyl alcohol, polyvinyl pyrrolidone, polyacrylic acid, poly ethylene oxides, tragacanth, sodium alginate, xanthan gum, gelatin, pectin, and the like and mixtures thereof. Any mixture of these bio-adhesive polymers may be used to obtain suitable pharmaceutical compositions as defined herein.

In a preferred embodiment, the at least one bio-adhesive polymer is carboxymethyl cellulose sodium. In another preferred embodiment, said at least one bio-adhesive polymer is hyaluronic acid and/or a salt thereof. The at least one bio-adhesive polymer (third polymer) provides an additional synergy with the first and second polymers leading to an improved level of adhesion of pharmaceutical composition and an improved residence time at the desired site.

In one embodiment, the at least one bio-adhesive polymer is contained in an amount which ranges from about 0.005% to about 5% by weight with respect to the weight of the pharmaceutical composition, more preferably from about, 0.01% to about 2.0% by weight with respect to the weight of the pharmaceutical composition, still more preferably from about 0.05% to about 0.1% by weight with respect to the weight of the pharmaceutical composition.

In a preferred embodiment, the at least one bio-adhesive polymer is contained in an amount of about 0.005% or about 0.01% and preferably about 0.05% by weight with respect to the weight of the pharmaceutical composition.

In one embodiment, the active agent present in the pharmaceutical composition is Rifamycin SV. The Rifamycin SV is contained in an amount which ranges from about 0.01% to about 10% by weight with respect to the weight of the pharmaceutical composition, preferably from about 0.1% to about 5% by weight with respect to the weight of the pharmaceutical composition, more preferably from about 0.25% to about 3% by weight with respect to the weight of the pharmaceutical composition. In a preferred embodiment, Rifamycin SV is contained in an amount of about 0.1% or about 0.2 % or about 0.25% or about 0.3% or about 0.4% or about 0.5% or about 1% or 2.5% and more preferably about 0.5% or 1.0% by weight with respect to the weight of the pharmaceutical composition. In one preferred embodiment, Rifamycin SV is contained in an amount of about 0.5% by weight with respect to the weight of the pharmaceutical composition. In another preferred embodiment, Rifamycin SV is contained in an amount of about 1.0% by weight with respect to the weight of the pharmaceutical composition. In one embodiment, the dosage strength of Rifamycin SV in the pharmaceutical composition of the present invention is established in order to achieve a local concentration of Rifamycin SV in the site of action exceeding its Minimum Inhibitory Concentration (MIC) on the specific bacterial population commonly localized therein. The MIC is defined as the lowest concentration expressed in mg/L (equivalent to µg/mL) of an antimicrobial or antibacterial active agent that is bacteriostatic, i.e., prevents the visible growth of bacteria. MICs are used to evaluate the antimicrobial efficacy of various compounds by measuring the effect of decreasing concentrations of antibiotic over a defined period in terms of inhibition of microbial population growth. MIC is generally regarded as the most basic laboratory measurement of the activity of an antimicrobial agent against an organism. Because a lower MIC value indicates that less of the drug is required in order to inhibit growth of the organism, drugs with lower MIC values are more effective antimicrobial or antibacterial active agents.

In one embodiment, the dosage of Rifamycin SV can be determined on the basis of the MIC for rifamycin SV on bacteria commonly found in and around the pouch (J-pouch or S-Pouch or W-Pouch) or ileostomy surgery (K-Pouch). In one embodiment, the dosage of rifamycin SV could be selected from within the MIC values for the commonly found bacteria. The dosage of Rifamycin SV can be a dose that kills all of the bacteria causing the pouchitis. In one embodiment, the pharmaceutical composition could be prepared to contain this dosage of Rifamycin SV and stored for future use. In another embodiment, the pharmaceutical composition could be prepared without the active agent and could be stored until usage. A suitable dosage of rifamycin SV, as described herein above, can thereafter be added to such pharmaceutical composition prior to administration.

In one embodiment, Rifamycin SV is fully dissolved in the pharmaceutical composition of the present invention. In another embodiment, Rifamycin SV is partly dissolved and partly in solid suspension in the pharmaceutical composition of the present invention. In one some embodiments, the pharmaceutical composition may contain one or more pharmaceutically acceptable excipients. According to the present invention, said pharmaceutically acceptable excipients may be selected in the group comprising: diluents, stabilizers, preservatives, anti-oxidants, buffers and other ingredients that are appropriate to the nature, composition and mode of administration of the pharmaceutical composition described herein. The types and amounts of pharmaceutically acceptable excipients that can be used in the pharmaceutical composition can be readily determined by the skilled artisan. In one embodiment, the pharmaceutical composition contains at least one anti-oxidant as a pharmaceutically acceptable excipient. In another embodiment, the pharmaceutical composition containing the at least one anti-oxidant further comprises at least one preservative as a further pharmaceutically acceptable excipient. Anti-oxidants and preservatives which are pharmaceutically acceptable are well known to the skilled artisan.

In one embodiment, the pharmaceutical composition described herein can be used to deliver Rifamycin SV directly in the targeted rectosigmoid region of the large intestine by direct, topical administration. In one embodiment, the pharmaceutical composition is suitable for rectal administration. In one embodiment, the pharmaceutical composition is in the form of an enema. In one embodiment, the pharmaceutical composition is a liquid in the form of a solution, suspension, foam, emulsion or microemulsion; preferably the pharmaceutical composition is in form of a solution and more preferably an aqueous solution.

In one embodiment, the pharmaceutical composition of the invention is in the form of an enema and its individual content per unit is in the range between 50 mL and 100 mL. In some embodiments, the individual content per unit of the pharmaceutical compositions of the invention in form of enema is 50 mL, or 60 mL, or 70 mL, or 80 mL, or 90 mL, or 100 mL. In a preferred embodiment, the individual content per unit of the pharmaceutical compositions of the invention in the form of an enema is 50 mL. In another preferred embodiment, the individual content per unit of the pharmaceutical compositions of the invention in form of enema is 60 mL. In further another preferred embodiment, the individual content per unit of the pharmaceutical compositions of the invention in form of enema is 100 mL.

As such, the pharmaceutical compositions of the present invention are intended for the treatment of diseases affecting the distal part of the large intestine, more specifically the sigmoid colon and rectum. In some embodiments, such diseases affecting the distal part of the large intestine are proctitis and/or distal ulcerative colitis. In another embodiment, the pharmaceutical composition can be administered directly by rectal administration, such as by the use of an enema, to cavities created surgically, from disease or resulting from injury. In one embodiment, the cavity is an ileal pouch (J-pouch or S-Pouch or W-Pouch) created by ileoanal anastomosis (J-Pouch or S-Pouch or W-Pouch) surgery or ileostomy surgery (K-Pouch). Thus, the pharmaceutical composition described herein is particularly suited for the treatment of pouchitis.

The pharmaceutical composition is prepared by serially adding the components of the pharmaceutical composition to water with stirring until complete dissolution or a homogeneous mixture is obtained eventually having the Rifamycin SV partially suspended as more fully described in the Examples below.

In a preferred embodiment, the pharmaceutical composition comprises rifamycin SV as the active agent and a polymer mixture comprising at least one thermo-responsive polymer, at least one ion-sensitive polymer and at least one bio-adhesive polymer. In one embodiment, the at least one thermo-responsive polymer may comprise poloxamer 407, the at least one ion-sensitive polymer may comprise alginic acid or salts thereof, and the at least one bio-adhesive polymer may comprise sodium carboxymethylcellulose. In one preferred embodiment, the polymer mixture comprises poloxamer 407, sodium alginate and sodium carboxymethylcellulose.

In one embodiment, poloxamer 407 may be present in an amount of about 5 % to about 30% by weight with respect to the weight of the pharmaceutical composition, optionally in the range of about 10% to about 25%, with respect to the weight of the pharmaceutical composition or in the range of about 12% to about 18% with respect to the weight of the pharmaceutical composition, preferably about 16% or 15% or 14% by weight with respect to the weight of the pharmaceutical composition. In a preferred embodiment, poloxamer 407 may be present in an amount of about 16.5% by weight with respect to the weight of the pharmaceutical composition.

In one embodiment, sodium alginate may be present in an amount of about 0.005% to about 5% by weight with respect to the weight of the pharmaceutical composition, optionally in the range of about 0.001% to about 3.0% by weight with respect to the weight of the pharmaceutical composition, or in the range of about 0.05% to about 2.0% by weight with respect to the weight of the pharmaceutical composition, preferably 0.2% or 0.1% (w/w) with respect to the weight of the pharmaceutical composition.

In one embodiment, sodium carboxymethylcellulose may be present in an amount of about 0.005% to about 5% by weight with respect to the weight of the pharmaceutical composition, optionally in the range of about 0.001% to 2% by weight with respect to the weight of the pharmaceutical composition, or in the range of about 0.05% to about 0.1% by weight with respect to the weight of the pharmaceutical composition, preferably about 0.05% (w/w) with respect to the weight of the composition.

In one embodiment, the pharmaceutical composition may further contain at least one anti-oxidant. In one embodiment, the anti-oxidant may be potassium metabisulfite, ascorbic acid, or sodium ascorbate or mixtures thereof. In one embodiment, the anti-oxidant comprises these three anti-oxidants. In one embodiment, the pharmaceutical composition may further contain at least one preservative. In one embodiment, the preservative may be potassium sorbate, sodium benzoate or ethanol or mixtures thereof. In one embodiment, the preservative comprises ethanol or a mixture of potassium sorbate and sodium benzoate

In one embodiment the mixture of the polymers and the Rifamycin SV is a unique system in which the amount of the Rifamycin SV in soluble form as well as partially suspended form influences the rheological properties of the mixture of polymers. The rheological properties unexpectedly decrease with an increase of the amount of Rifamycin SV added to the pharmaceutical composition. In one embodiment, the pharmaceutical composition containing 0.5% by weight of Rifamycin SV has a qualitative/quantitative composition of the selected polymers which leads the starting of sol to gel transition at a temperature in between 27°C and 37°C. In one embodiment, the pharmaceutical composition containing 0.5% by weight of Rifamycin SV gelifies at body temperature (around 37°C) and in turn this allows to manage the product at room temperature as liquid form (patient's compliance) and to have a gel product at the body temperature (around 37°C) after administration by rectal route. The addition of Rifamycin SV into the mixture of polymers unexpectedly leads to the shift of the temperature switch between the modules G' and G'' of two degrees (2°C) compared to the vehicle (polymer mixture without Rifamycin SV or placebo) and decreases the intensity of the G' module compared to the vehicle. In one embodiment, the amount of Rifamycin SV added to the composition surprisingly shows an increase of G' modulus over time at 37°C (time dependency) not evident in the polymer mixture in absence of Rifamycin SV (vehicle). It is surprisingly found a time dependency of the pharmaceutical composition with Rifamycin SV with respect to vehicle composition as evidenced by G' curve. This surprisingly demonstrated that the addition of Rifamycin SV into the polymer mixtures provides the improved long-lasting effect of the gel overtime at the target site compared with the polymers mixture alone. Unexpectedly the Rifamycin SV interacts with the polymers mixture increasing the G' modulus overtime allowing the Rifamycin SV to remain embedded in the thin layer of the gel in tight contact with the site of action, and prolonging the duration of its pharmacological activity in situ strengthening the gel structure at the target site (see Example 7). In one embodiment, the pharmaceutical composition including Rifamycin SV totally preserves the MICs value of the Rifamycin SV tested alone as reported in the Examples below. Along with the improved in situ retention due to the enema vehicle, this maintenance of MICs allows for a longer efficacy of the locally administered drug in terms of antibiotic effect. In one embodiment, the pharmaceutical composition including Rifamycin SV shows an increased anti-inflammatory effect when compared with the same amount of Rifamycin SV tested alone. The skilled artisan would recognize that the increased anti-inflammatory action of pharmaceutical compositions containing Rifamycin SV of the present invention, as compared to the same amounts of the active substance Rifamycin SV tested alone, are unexpected and unpredictable. The non-active components of the pharmaceutical composition of the present invention, as disclosed hereinabove, are constituted by known pharmaceutical excipients not endowed of any anti-inflammatory activity. As evidence of this lack of anti-inflammatory activity, there stand the results of the anti-inflammatory test conducted on the pharmaceutical composition of the invention not containing the active substance (also referred to as "the vehicle"). As expected, such test confirmed the lack of any anti-inflammatory activity of the vehicle alone, not including Rifamycin SV, given the known lack of pharmacological activity of the pharmaceutical excipients of the invention disclosed herein. The skilled artisan would recognize that a synergistic effect or an advantageous and beneficial interaction there might exist between the vehicle of the present invention and the Rifamycin SV active substance, that enhances the intrinsic anti-inflammatory activity of the Rifamycin SV active substance. As such, the pharmaceutical compositions of the present invention, formulated as in situ gelling enema containing Rifamycin SV, exert an anti-inflammatory activity which is higher than the Rifamycin SV alone, and are of particular utility in treating, ameliorating, reducing the severity of, inducing, maintaining the remission of, and/or slowing the progression of gastrointestinal diseases with an inflammatory component, such as pouchitis, or proctitis, or ulcerative colitis.

The pharmaceutical composition of the invention, comprising Rifamycin SV and the three polymers as above disclosed, is thus able to provide a long-lasting effect and enhanced adhesion of Rifamycin SV at the site of action (distal part of the gastro-intestinal tract and/or the artificial cavity surgically created ileal pouch), avoiding the quick evacuation of the composition with the significant loss of activity. Moreover, Rifamycin SV showed a dual therapeutic effect, combining an antibiotic action with an anti-inflammatory effect, providing a complete therapeutic system with a broad spectrum of action. Up to date there is no treatment able to combine a dual therapeutic effect together with a higher permanence time at the site with an improved long-lasting adhesion.

The anti-inflammatory efficacy, together with the antibiotic effect of the Rifamycin SV formulated in the pharmaceutical composition of the invention comprising the three polymer mixtures leads to an effect in treating, ameliorating, reducing the severity of, inducing, maintaining the remission of, and/or slowing the progression of gastrointestinal diseases with an inflammatory and/or infectious component. Rifamycin's short-term but immediate clinical benefit of reducing inflammation, its clinical benefit of bacterial infection eradication, and minimal absorption at the intestinal level, renders the composition of the invention advantageous treatments for all 3 categories of pouchitis: antibiotic-responsive, antibiotic-dependent, and antibiotic-resistant, or any other gastrointestinal diseases with an inflammatory and/or infectious component.

Rifamycin SV formulated into the in situ gelling formulation according to the invention is unique.

The Rifamycin SV minimal systemic absorption in the gastrointestinal tract is a distinctive key element when compared to other antibiotics currently used in pouchitis treatment (e.g., ciprofloxacin and metronidazole). These treatments are administered orally and their systemic absorption may more frequently lead to antibiotic-resistant strains over time, along with long-term adverse effects due to the systemic exposure and biodistribution. Since patients with pouchitis often suffer more than 2-3 acute episodes per year, the use of a non-adsorbable antibiotic such as Rifamycin may offer a crucial advantage for these frequent relapsing patients. Additionally, serious adverse reactions that have been reported in patients treated with different antibiotics, such as, for example, convulsive seizures and peripheral neuropathy. Thus, in such patients, the use of a non-absorbable antibiotic with a favorable safety profile as Rifamycin SV may offer a safer alternative to the current standard of care treatments (such as, for example, ciprofloxacin and metronidazole).

**DEFINITIONS**

References in the specification to "one embodiment", "an embodiment", "one aspect", "an aspect" and similar indicate that the described embodiment or aspect may include a particular aspect, feature, structure or characteristic. Moreover, such phrases may, but do not necessarily, refer to the same embodiment or aspect referred to in other portions of the specification. Further, when a particular aspect, feature, structure or characteristic is described in connection with an embodiment or aspect, it is within knowledge of a person skilled in the art to affect or connect said aspect, feature, structure or characteristic with other embodiment or aspect, whether or not explicitly described. The singular forms "a", "an" and "the" include plural references unless the context clearly dictates otherwise. Thus, for example, a reference to "a compound" includes a plurality of such compounds. It is further noted that the claims may be drafted to exclude any optional element. As such, this statement is intended to serve as antecedent basis for the use of exclusive terminology, such as "solely", "only", and the like, in connection with the recitation of claims elements or use of a "negative" limitation.

The term "and/or" means anyone of the items, any combination of the items, or all the items with which this term is associated.

The terms "comprising", "having", "including" and "containing" are to be construed as open-ended terms (i.e. meaning "including, but not limited to") and are to be considered as providing support also for terms as "consist essentially of", "consisting essentially of', "consist of' or "consisting of".

The terms "consist essentially of", "consisting essentially of' are to be construed as a semi-closed terms, meaning that no other ingredients which materially affects the basic and novel characteristics (and optionally physiologically acceptable excipients and/or adjuvants) of the invention are included.

The terms "consists of', "consisting of' are to be construed as a closed term.

The term "polymer mixture" is intended to mean the pharmaceutical composition without the active agent. Thus, in its simplest usage, the "polymer mixture" comprises water or other suitable carrier for the formulation of an enema, the at least one thermo-responsive polymer, the at least one ion-sensitive polymer and the at least one bio-adhesive polymer, as well as any excipients described herein.

Unless indicated otherwise herein, the term "about" is intended to include values, e.g. weight percentages, proximate to the recited range that are equivalent in terms of the functionality of the individual ingredient, the composition, or the embodiment.

A person skilled in the art will recognize that, for any and all purposes, particularly in terms of providing a written description, all ranges recited herein also encompass any and all possible sub-ranges and combinations of sub-ranges thereof, as well as the individual values making up the range, particularly integer values. A recited range includes each specific value, integer, decimal, or identity within the range.

A person skilled in the art will recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of anyone or more of members of a recited group. Accordingly, provisos may apply to any of the disclosed categories or embodiments whereby anyone or more of the recited elements, species, or embodiments, may be excluded from such categories or embodiments, for example, as used in an explicit negative limitation.

The term "alleviating" refers to relieving the symptoms and/or manifestations of inflammatory and/or degenerative diseases of the gastrointestinal tract.

The term "reducing" refers to decreasing the extent of the damage caused by inflammatory and/or degenerative diseases of the gastrointestinal tract or to decreasing clinical signs or symptoms associated with such damage.

The "viscosity" defines the resistance of a liquid or semisolid against flow. The flow of liquids or semisolids is described by viscosity, or, more precisely, by shear viscosity $\eta$. The shear viscosity of a fluid expresses its resistance to shearing flows, where adjacent layers move parallel to each other with different speeds. Common units of measurement of viscosity are the pascal-second (Pas), the poise (P) and cP (centipoises).

"Modulus G'" refers to elastic or storage modulus obtained in dynamic regimen. An elastic modulus (also known as modulus of elasticity) is a number that measures an object or substance's resistance to being deformed elastically (i.e., non-permanently) when a stress is applied to it. The elastic modulus of an object is defined as the slope of its stress-strain curve in the elastic deformation region.

"Body temperature" refers to the level of heat produced and sustained by the body processes. Heat is generated within the body through metabolism of nutrients and lost from the body surface through radiation, convection, and evaporation of perspiration. Heat production and loss are regulated and controlled in the hypothalamus and brainstem. Normal adult body temperature, as measured orally, is 37 °C even though little variations are normally recorded throughout the day.

"Room temperature" (RT) is generally defined as the ambient air temperature in whatever environment being used for a given procedure. More specifically, it is defined as 20-25 °C., as some ambient temperatures, by nature, do not fall within this range. Generally, protocols calling for steps to be performed at RT require that temperatures do not fall below 18 °C, and do not exceed 27 °C.

"MIC" refers to Minimum Inhibitory Concentration and it is defined as defined as the lowest concentration expressed in mg/L (equivalent to $\mu$g/mL) of an antimicrobial ingredient or agent that is bacteriostatic (prevents the visible growth of bacteria)

"DoE" refers to Design of Experiments and it is defined as a statistical and mathematical tool to perform the experiments in a systematic way and analyses data efficiently.

"Rifamycin SV" refers to Rifamycin, Rifamycin sodium salt defined to the CAS N° 14897-39-3.

**EXAMPLES**

The following examples are included for purpose of illustration of certain aspects and aspects of the invention, and are not intended to limit the invention.

Example 1

Simplex Centroid Design with and without Rifamycin

A pharmaceutical composition comprising Rifamycin SV and a polymer mixture comprising Poloxamer, Sodium Alginate and Carboxymethylcellulose Sodium was prepared. The pharmaceutical composition is intended for the application to the Pouch, distal Intestine and rectum. A DoE approach, "simplex centroid design", is used to better understand the interaction and ratio between the mixture of polymers (vehicle) with and without Rifamycin SV. The response variables considered are:

1) polymer mixture viscosity at room temperature (25 °C), functional to an easy administration;

2) viscoelastic behavior after administration, functional to the polymer mixture's sustained release action at the application site; and

3) mucoadhesion at 37 °C, functional to maintenance in-situ of the polymer mixture after administration.

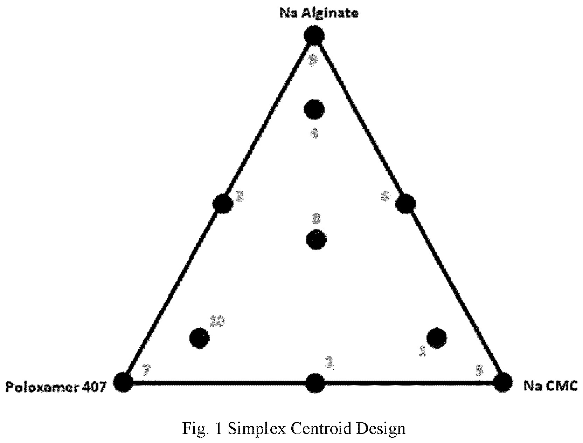

Table 1 and Fig. 1 report factor space and experimenting points of a simplex centroid design.

TABLE 1

| Fractional Mixture Composition Corresponding to the Characteristic Points of the Simplex Centroid Mixture | | | |
|---|---|---|---|
| **Points of Scheffè triangle** | **Sodium alginate % w/w** | **Poloxamer 407 % w/w** | **Na CMC % w/w** |
| Vehicle (Run) 1 | 0.17 | 5 | 0.67 |
| Vehicle (Run) 2 | // | 15 | 0.5 |

(continued)

| Fractional Mixture Composition Corresponding to the Characteristic Points of the Simplex Centroid Mixture | | | |
|---|---|---|---|
| Points of Scheffè triangle | Sodium alginate % w/w | Poloxamer 407 % w/w | Na CMC % w/w |
| Vehicle (Run) 3 | 0.5 | 15 | // |
| Vehicle (Run) 4 | 0.67 | 5 | 0.17 |
| Vehicle (Run) 5 | // | // | 1 |
| Vehicle (Run) 6 | 0.5 | // | 0.5 |
| Vehicle (Run) 7 | // | 30 | // |
| Vehicle (Run) 8 | 0.33 | 10 | 0.33 |
| Vehicle (Run) 9 | 1 | // | // |
| Vehicle (Run) 10 | 0.17 | 20 | 0.17 |

The mixture design shown in Fig. 1 includes: 3 single mixture components, corresponding to the triangle corners; 3 binary combinations, graphically represented as points along the triangle edges; 1 ternary combination, that overlaps with the center of the triangle, and 3 ternary combination within the area inside the triangle [Brereton, 2003]. Two parallel centroid mixture design have been performed one with the addition of active ingredient (Rifamycin SV 0.5% w/w, equivalent to 5 mg/g) and one without the addition of active ingredient, following the scheme of Table 1 in order to obtain the significant points of the simplex centroid design. The reason of such duplication of the design (with and without Drug Substance) was to verify whether the Rifamycin specifically interacts with the gelling properties of the selected polymers mixture and, if any chemical and/or physical interferences occur, whether this interaction leads to improved or worsened rheological properties.

The 10 points polymer mixtures of Table 1 (with and without Rifamycin SV) were characterized for viscosity at increasing shear rates (0,1 - 300 s$^{-1}$) by means of a rotational rheometer (Kinexus Pro+) at 25 °C and 37 °C. All the polymer mixtures (with and without Rifamycin SV) were subjected to viscoelastic measurements (oscillation test) at both 25 °C and 37 °C by means of the above-mentioned rotational rheometer (Kinexus Pro+) and to mucoadhesion characterization. To characterize the muco-adhesion properties, all the vehicles with Rifamycin were assessed at 37 °C by means of a TA-XT plus Texture Analyzer. Such a test measures the force of detachment (Fmax, mN) and work of adhesion (AUC, mN.mm) necessary to separate a vehicle layer from a filter disc soaked with 8% w/w suspension of a commercial gastric mucin (type II, Sigma, I) compared to purified water as blank solution. Data were also normalized between mucin and blank in order to better understand the adhesion of the formulations comparing the two substrates. The normalized Force and Work of adhesion (AUC) were calculated according to the following equations:

$$\Delta Fmax/\ Fmax\ blank = (Fmax\ mucin - Fmax\ blank)\ /\ Fmax\ blank$$

and

$$\Delta\ AUC\ /\ AUC\ blank = (AUC\ mucin - AUC\ blank)\ /\ AUC\ blank$$

Where Fmax mucin and AUC mucin are respectively force and work of adhesion obtained in presence of gastric mucin dispersion at 8% w/w and Fmax blank and AUC blank are respectively force and work of adhesion obtained from blank measurement.

The following response variables were measured:

1) viscosity of the polymer mixture as is at 25 °C and 37 °C, at 10 s$^{-1}$ shear rate;
2) as for viscoelatic measurements:

1) Storage elastic modules (G');
2) Loss viscous modules (G");
3) loss tangent (Tan delta), calculated as ratio between loss module, which is an index of viscous behavior (G") and storage elastic (G') moduli;

as for muco-adhesion measurements:

1) maximum force of detachment (Fmax)
2) work of adhesion (AUC)
3) Normalized data of force (Δ Fmax / Fmax blank)
4) Normalized data of work (Δ AUC / AUC blank).

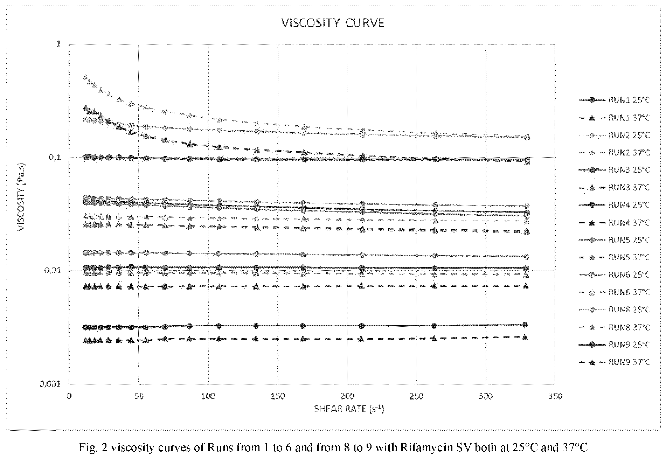

Each response variable can be related to polymer mixture composition by means of a suitable mathematical model. To this purpose, experimental data were subjected to multiple linear regression analysis, testing a series of models (linear, quadratic and special cubic) [Draper and Smith (1981)]. To determine the best fitting model for each response variable, a statistical analysis (ANOVA) was performed by means of a statistical software package (Minitab® 19, 2020).
Viscosity curves were obtained for each run with or without Rifamycin SV and at 25 °C and 37 C. Representative viscosity curves at 25°C and 37°C of run with Rifamycin, are shown in Fig. 2. The comparison of viscosity data obtained from Runs 1 to 10 (Placebo vs Rifamycin SV formulations) show that:

- Run 7 and Run 10 viscosity data are similar both in Placebo and Rifamycin formulations showing an higher viscosity values at 10 s$^{-1}$ (100 time higher than the others at 25 °C and 37 °C) and there is no difference between viscosity curves at 25 °C and 37 °C, likely due to the higher concentration of Poloxamer meaning that no transition occurs with temperature;
- Run 2 and Run 3 containing 15% of Poloxamer in the formula, show a difference between 25 °C to 37 °C viscosity profiles. Both formulations show a Newtonian behavior at 25 °C while, a pseudoplastic behavior at 37 °C. Furthermore, viscosity values at 37 °C, at 10 s$^{-1}$, are higher compared to values at 25 °C. The same behavior is shown in each sample runs for Placebo and Rifamycin SV batches, even if Rifamycin SV batches show a decrease of viscosity values in comparison with the corresponding vehicle runs.

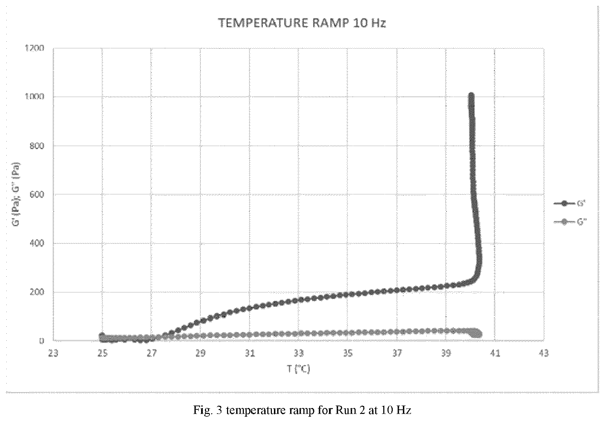

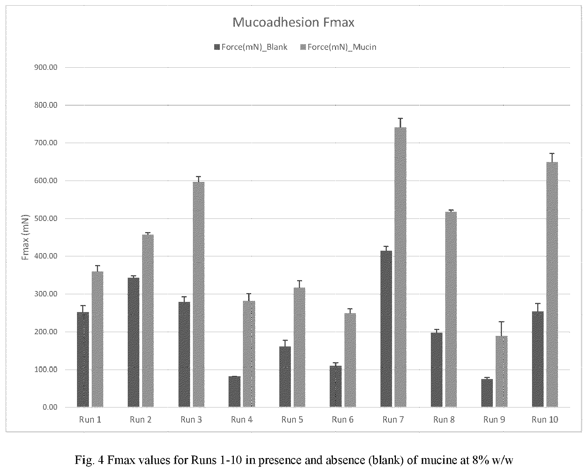

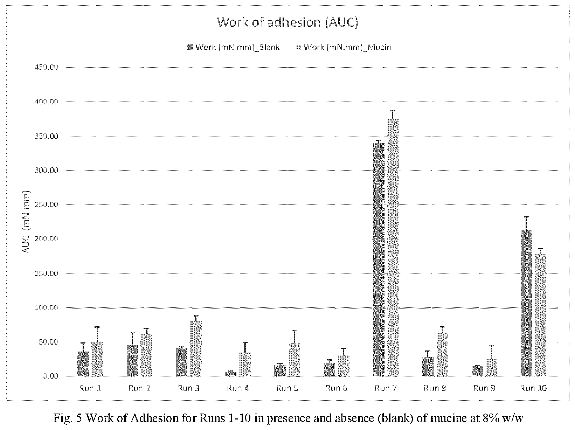

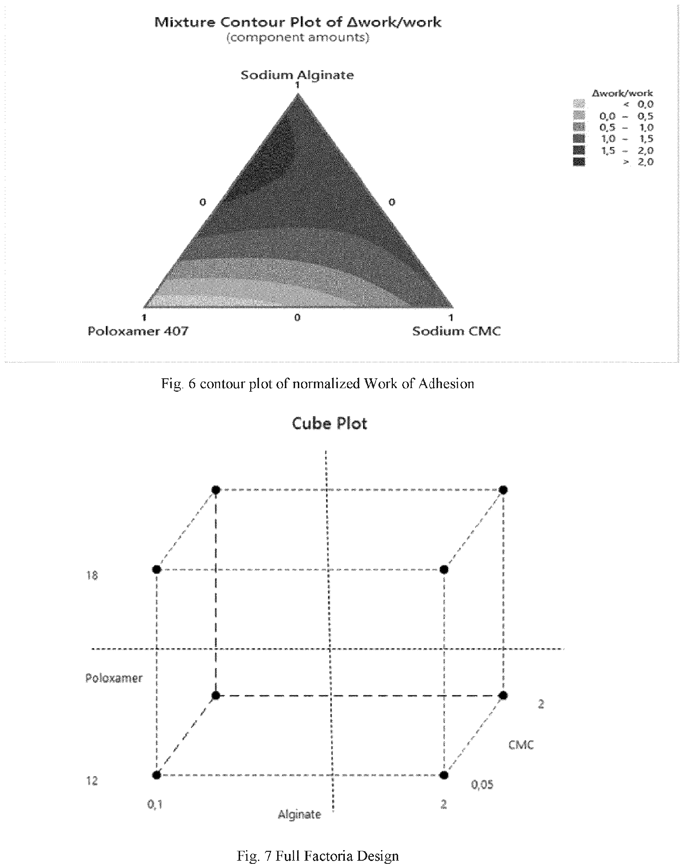

Considering the viscoelastic data, Run 7 and Run 10 (both with concentration of Poloxamer > 20%) do not show a switch of modules G' and G" from 25 °C to 37 °C. In fact, both Run 7 and Run 10 viscosity curves at 25°C show higher values in comparison with the other runs. On the other hand, Run 2 and Run 3 show a switch of modules G' and G" around 28 °C. Fig. 3 shows the temperature ramp for Run 2.
The muco-adhesion results in terms of Fmax of the runs containing Rifamycin SV show that Run 3, 4, 7, 8 and Run 10 show an increase in mucoadhesive properties following the interaction with mucin as shown in Fig. 4. Work of adhesion results (AUC) confirmed the results obtained by Fmax values, mostly on run 7 and 10 that presented high values in both the substrates, meaning high interaction in both conditions. This interaction is due to high concentration of poloxamer in both runs. See Fig. 5. Normalized results permit an easier understanding of the data: combination of the three polymers led to high muco-adhesion as observed in Run 4. Fig. 6 is illustrative of the contour plot of normalized Work (AUC) extrapolated from Mixture Design. Highest values of Work of adhesion fallen in the region with combination of all of three polymers (dark green) with values approximately below 15% of Poloxamer 407, approximately above 0.5 % of Sodium Alginate and approximately below 0.17% of Sodium CMC.
Through the outcomes of this first Mixture Design a region of optimization for the polymer mixture was defined to be approximately between 14% and 15% w/w of Poloxamer 407, approximately between 0.4% and 0.1% of Sodium Alginate and approximately between 0.4 and 0.01 of Sodium CMC. A further 3-factors Full Factorial Design was carried out by using selected polymer ranges, in order to better define the optimum of the formulation composition.

Example 2

FULL FACTORIAL

Identification of the Optimal Quantitative Composition of the Polymer Mixture

The pharmaceutical composition is intended for the application to the J pouch, distal intestine and rectum. A DoE approach, "Full Factorial design", was used to identify the optimal quantitative composition of the polymer mixture. The response variables considered were:

1) polymer mixture viscosity at room temperature (25 °C), functional to an easy administration
2) viscoelastic behavior after administration, functional to the polymer mixture's sustained release action at the application site; and
3) muco-adhesion at 37 °C, functional to maintenance in-situ of the polymer mixture after administration.

Table 2 and Figure 7 report factor space and experimenting points of a Face Centered Full Factorial Design.

TABLE 2

| Fractional Mixture Composition Corresponding to the Characteristic Points of the Full Factorial Design | | | |
|---|---|---|---|
| Points of cube plot | SODIUM ALGINATE % w/w | POLOXAMER 407 % w/w | SODIUM CMC % w/w |
| Vehicle (Run) 1 | 2,000 | 18,000 | 2,000 |
| Vehicle (Run) 2 | 0,100 | 12,000 | 0,050 |
| Vehicle (Run) 3 | 0,100 | 12,000 | 2,000 |
| Vehicle (Run) 4 | 2,000 | 12,000 | 0,050 |
| Vehicle (Run) 5 | 0,100 | 18,000 | 0,050 |
| Vehicle (Run) 6 | 0,100 | 18,000 | 2,000 |
| Vehicle (Run) 7 | 2,000 | 12,000 | 2,000 |
| Vehicle (Run) 8 | 2,000 | 18,000 | 0,050 |
| Vehicle (Run) 9 | 0,100 | 15,000 | 1,025 |
| Vehicle (Run) 10 | 2,000 | 15,000 | 1,025 |
| Vehicle (Run) 11 | 1,050 | 12,000 | 1,025 |
| Vehicle (Run) 12 | 1,050 | 18,000 | 1,025 |
| Vehicle (Run) 13 | 1,050 | 15,000 | 0,050 |
| Vehicle (Run) 14 | 1,050 | 15,000 | 2,000 |

The DoE set has three factors that correspond to the three polymers. Each factor has two levels, containing also axial point (Face Centered). Each factor has a lower level (-1), a higher level (+1) and an axial one (0).

The 14 points polymer mixtures of Table 2 (with Rifamycin SV) are characterized for viscosity at increasing shear rates (0,1 - 300 s-1) by means of a rotational rheometer (Kinexus Pro+) at 25 C. All the polymer mixtures (with and without Rifamycin SV) are subjected to viscoelastic measurements (oscillation test) at both 25°C and 37°C by means of the above-mentioned rotational rheometer (Kinexus Pro+) and to muco-adhesion characterization.

The following response variables are measured:

1) viscosity of the polymer mixture as is at 25 °C and 37 °C, at 10 s-1 shear rate;
2) as for viscoelastic measurements:

   1) Storage elastic modules (G');
   2) Loss viscous modules (G");
   3) Loss tangent (Tan delta) calculated as ratio between loss module, which is an index of viscous behavior (G") and storage elastic (G') moduli.

as for muco-adhesion measurements:

   1) maximum force of detachment (Fmax)
   2) work of adhesion (AUC)
   3) Normalized data of force ($\Delta$ Fmax / Fmax blank)
   4) Normalized data of work ($\Delta$ AUC / AUC blank)

Each response variable can be related to polymer mixture composition by means of a suitable mathematical model. To this purpose, experimental data were subjected to multiple linear regression analysis, testing a series of models (linear, quadratic and special cubic) [Draper and Smith (1981)]. To determine the best fitting model for each response variable, a statistical analysis (ANOVA) was performed by means of a statistical software package (Minitab[®] 19, 2020).

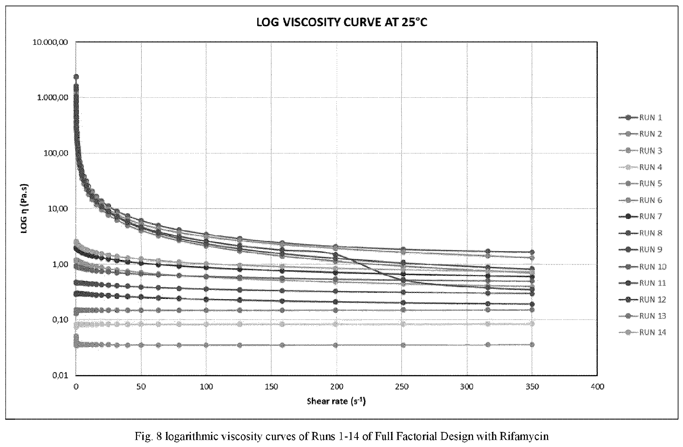

Viscosity curves were obtained for each run with Rifamycin SV at 25 °C. Representative viscosity curves at 25 °C are shown in Fig. 8. The comparison of viscosity data obtained from Runs 1 to 14 showed that Run 2 with all polymers at lower levels, presented the least viscous behavior; on the contrary, run 1, which has a higher level for all the three polymers, is the most viscous.

From the experiments, it appears that lower levels of Poloxamer 407 and Sodium CMC have an impact on the vehicle viscosity at 25°C and 10 s-1, in fact vehicles with the lowest level of these two polymers are those characterized by low viscosity (e.g. run 4). However, the addition of Sodium Alginate to these vehicles, sharply increases viscosity.

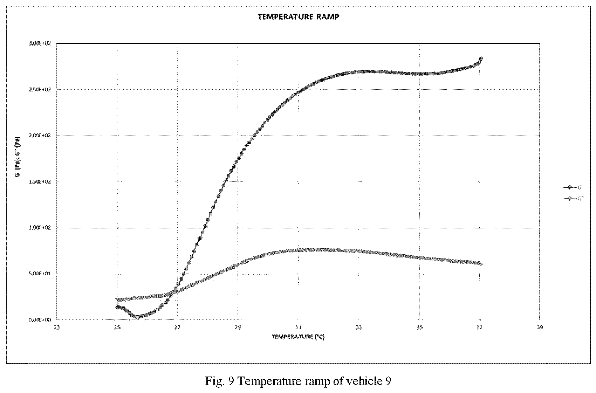

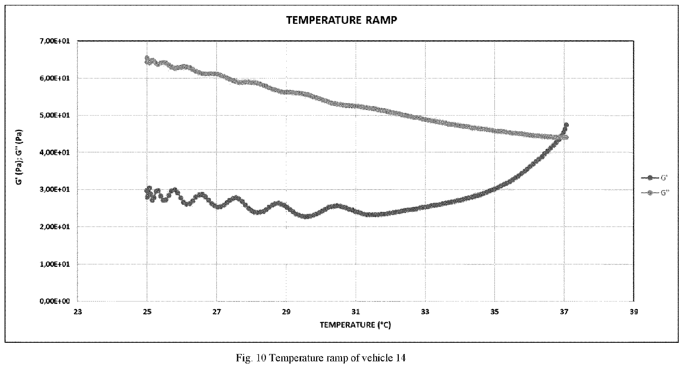

Considering the viscoelastic data, it was evaluated that Poloxamer is the primary responsible of formulation gelation at physiological conditions due to its thermogelation properties. When Poloxamer 407 concentration is at 12% w/w, thermogelation did not occur in most of the studied runs, with exception of Run 2. On the other hand, when the concentration was 18% w/w, the formulation presented solid-like properties already at room temperature. When Poloxamer 407 was at its axial point (15% w/w), a switch of modules G' and G" occurred into a range of temperature between 27 and 37 °C depending on the formulation; the switch gave to the formulations a solid-like behavior. Figure 9 and 10 give an example of this tendency.

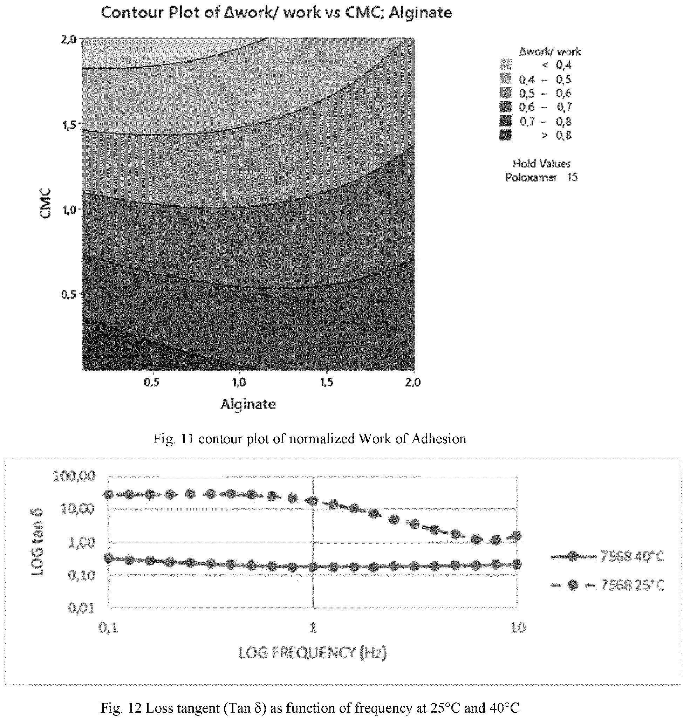

The muco-adhesion results in terms of Work of adhesion of both series confirmed data obtained by muco-adhesion force. Normalized results of Fmax and Work of Adhesion (AUC), were analyzed by the Full Factorial Design and, from Pareto chart, it was established that the most statistically significant factor was Poloxamer 407 and the mixture of the three polymers. Furthermore, contour plot of normalized Force and Work were extrapolated from Full Factorial Design. High values of Force and Work of adhesion were observed with value of Poloxamer 407 around 15%, approximately below 1.0% of Sodium Alginate and approximately below 0.5% of Sodium CMC. Fig. 11 shows a good Work of Adhesion when Poloxamer P 407 is around 15%, Sodium Alginate is approximately below 1.0 % and Sodium CMC is approximately below 0.5%.

Example 3

Batch 7568

Batch 7568 aqueous solution was prepared as follows.

| COMPONENTS | %(W/W) |
|---|---|
| Rifamycin SV | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Potassium Sorbate | 0.100 |
| Sodium Benzoate | 0.100 |
| Purified Water | Up to 100 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then Potassium Sorbate is added to the mixture above under stirring until a complete dissolution is achieved. Then Sodium Benzoate is added to the mixture above under stirring until a complete dissolution is achieved. Then the pH of the solution is checked and brought it to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then the pH is checked and brought it to pH 7.0, if necessary. The mixture is brought to final weight adding Purified water.

The following characteristics have been considered:

1) viscosity of the composition after temperature increases from 25 °C to 37 °C, at 10Hz;

2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp at 10 Hz shows a switch between G' and G" modules starting from about 27 °C up to 29 °C. This crossover is associated with an increased elasticity of the sample.

Loss tangent (Tan $\delta$) as a function of frequency at 25 °C and 40 °C was determined. Tan $\delta$ < 1 shows a gel-like behaviour meaning a prevalence of the elastic behaviour on the viscous one at 40°C. Meanwhile at 25 °C, tan $\delta$ > 1 shows a liquid-like behaviour confirming the sol-gel transition. Fig. 12 provides a graph of loss of tangent (tan $\delta$) for Batch 7568. This graph is representative of other loss of tangent.

Example 4

Batch 7569

Batch 7569 aqueous solution was prepared as follows.

| COMPONENTS | %(W/W) |
|---|---|
| Rifamycin SV | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Ethanol | 1.000 |
| Purified Water | Up to 100 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

The following characteristics have been considered:

1) viscosity of the composition after temperature increases from 25 °C to 37 °C, at 10Hz;

2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp at 10 Hz shows a switch between G' and G" modules starting from about 26 °C up to 29 °C. This crossover is associate to an increased elasticity of the sample.

Tan δ < 1 show a gel-like behaviour meaning a prevalence of the elastic behaviour on the viscous one at 40 °C. Meanwhile at 25 °C tan δ > 1 show a liquid-like behaviour confirming the sol-gel transition.

Example 5

Batch 7572

Batch 7572 aqueous solution was prepared as follows.

| COMPONENTS | %(W/W) |
|---|---|
| Rifamycin SV | 0.500 |
| Poloxamer 407 | 16.000 |
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Ethanol | 2.000 |
| Purified Water | Up to 100 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

The following characteristics have been considered:

1) viscosity of the composition after temperature increase from 25 °C to 37 °C, at 10Hz;
2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp at 10 Hz shows a switch between G' and G" modules starting from about 26 °C up to 31 °C. This crossover is associate to an increased elasticity of the sample.

Tan δ < 1 show a gel-like behaviour meaning a prevalence of the elastic behaviour on the viscous one at 40 °C. Meanwhile at 25 °C tan δ > 1 show a liquid-like behaviour confirming the sol-gel transition.

Example 6

Batch 7567

Batch 7567 aqueous solution was prepared as follows.

| COMPONENTS | %(W/W) |
|---|---|
| Rifamycin SV | 0.500 |
| Poloxamer 407 | 14.00 |
| Gellan Gum | 0.300 |
| Hydroxypropyl cellulose | 0.400 |
| Potassium metabisulfite | 0.012 |
| Ascorbic acid | 0.043 |
| Sodium Ascorbate | 0.048 |
| Purified Water | Up to 100 |

In a suitable vessel provided with a stirrer, Purified water (95% of the total amount) is loaded; then, Hydroxypropyl cellulose is added to the mixture above under stirring until a homogeneous mixture is obtained. Then Ascorbic acid and Sodium Ascorbate are added under stirring until complete dissolution is achieved. Then Potassium Metabisulfite is added to the mixture above under stirring until a homogeneous mixture is achieved. Then the solution is cooled at a temperature ranging between 5 °C and 20 °C; then, poloxamer is added until a homogeneous mixture is achieved. The mixture is kept under stirring until a complete dissolution is achieved and then heated up to room temperature. Then check the pH of the solution and bring it to pH range 6.0-6.5 if needed. Then, Rifamycin SV is added to the mixture above under stirring until complete dissolution is achieved. Then Gellan gum is added to the mixture above under stirring until a homogeneous mixture is achieved. Then the pH is checked and brought to pH 7.0, if necessary. The mixture is brought to final weight adding Purified water.

The following characteristics have been considered:

1) viscosity of the composition after temperature increase from 25 °C to 37 °C, at 10Hz;

2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli

The temperature ramp at 10 Hz doesn't show a switch between G' and G" modules probably started before 25 °C up to 30 °C. However, G' module is much higher than G".

Tan $\delta$ < 1 show a gel-like behaviour meaning a prevalence of the elastic behaviour on the viscous one both at 25 °C and 40 °C. Tan $\delta$ < 1 shows that no sol-gel transition is observed in this temperature range.

Example 7

Batch 7570

Batch 7570 aqueous solution was prepared as follows.

| COMPONENTS | % w/w |
|---|---|
| Rifamycin SV | 0.500 |
| Poloxamer 407 | 16.000 |

(continued)

| COMPONENTS | % w/w |
|---|---|
| Sodium Alginate | 0.200 |
| Carboxymethylcellulose Sodium | 0.050 |
| Potassium metabisulfite | 0.012 |
| Ascorbic acid | 0.043 |
| Sodium Ascorbate | 0.048 |
| Purified Water | up to 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then Potassium Metabisulfite is added to the mixture above under stirring until a homogeneous mixture is achieved. Then the pH of the solution is checked and brought t to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then check the pH and bring it to pH 7.0, if necessary. The mixture is brought to final weight adding Purified water.

The following characteristics have been considered:

1) viscosity of the composition after temperature increase from 25 °C to 37 °C, at 10Hz;
2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp at 10 Hz shows a switch between G' and G" modules starting from about 26 °C up to 28 °C. This crossover is associated with an increased elasticity of the sample. The temperature ramp at 10 Hz of the vehicle without Rifamycin SV shows a switch between G' and G" modules starting from about 24°C up to 26°C.

Tan $\delta$ < 1 show a gel-like behaviour meaning a prevalence of the elastic behaviour on the viscous one at 40 °C. Meanwhile at 25 °C tan $\delta$ > 1 show a liquid-like behaviour confirming the sol-gel transition.

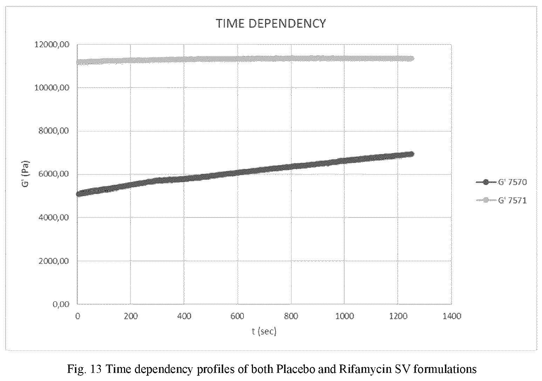

Rifamycin SV formulations and corresponding Placebo formulations were subjected to a constant frequency and shear stress for 20 minutes at constant temperature, 37 °C to evaluate if a time dependency occurs. Fig. 13.

It was surprisingly found that only the formulations containing Rifamycin SV present a time dependency meaning an increase of G' module over time. The time dependency confirms the increasing of the residence time of the drug at the site of action preventing the three-dimensional structure of the gel from being destroyed.

Example 8

Batch 7574

Batch 7574 aqueous solution was prepared as follows.

| COMPONENTS | % w/w |
|---|---|
| Rifamycin SV | 0.500 |
| Poloxamer 407 | 16.000 |
| Sodium Alginate | 0.200 |
| Carboxymethylcellulose Sodium | 0.050 |

(continued)

| COMPONENTS | % w/w |
|---|---|
| Ascorbic acid | 0.040 |
| Sodium Ascorbate | 0.045 |
| Ethanol | 1.000 |
| Purified Water up to | 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought it to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

The following characteristics have been considered:

1) viscosity of the composition after temperature increase from 25 °C to 37 °C, at 10Hz;
2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp shows a switch between G' and G" modules starting from about 26 °C up to 29 °C. This crossover is associate to an increased elasticity of the sample.

Tan $\delta$ < 1 show a gel-like behaviour meaning a prevalence of the elastic behaviour on the viscous one at 40 °C. Meanwhile at 25 °C tan $\delta$ > 1 show a liquid-like behaviour confirming the sol-gel transition.

Both Placebo and Rifamycin formulations were subjected to a constant frequency and shear stress for 20 minutes at constant temperature, 37 °C to evaluate if a time dependency occurs. It was surprisingly found that only the formulations containing Rifamycin present a time dependency meaning an increase of G' module over time. The time dependency confirms the increasing of the residence time of the drug at the site of action preventing the three-dimensional structure of the gel from being destroyed.

Example 9

Batch 7601

This batch corresponds to the optimized formulation extrapolated by the Full Factorial Design. Batch 7601 aqueous solution was prepared as follows.

| COMPONENTS | % w/w |
|---|---|
| Rifamycin SV | 0.500 |
| Poloxamer 407 | 13.600 |
| Sodium Alginate | 0.100 |
| Carboxymethylcellulose Sodium | 0.050 |
| Ascorbic Acid | 0.040 |
| Sodium Ascorbate | 0.045 |

(continued)

| COMPONENTS | % w/w |
|---|---|
| Ethanol | 1.000 |
| Purified Water up to | 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin is added SV to the mixture above under stirring until complete dissolution is achieved. Then the solution is cooled at a temperature ranging between 5°C and 20°C; then, Poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

The following characteristics has been considered:

1) viscosity of the composition after temperature increased from 25 °C to 37 °C, at 10Hz;
2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp shows a switch between G' and G" modules starting from 32°C up to 35°C. This crossover is associated with an increased elasticity of the sample.

Tan $\delta$ < 1 shows a gel-like behaviour meaning a prevalence of the elastic behaviour on the viscous one at 37°C. Meanwhile at 25°C tan $\delta$ > 1 shows a liquid-like behaviour confirming the sol-gel transition.

The formulation was subjected to a constant frequency and shear stress for 20 minutes at constant temperature (37°C), to evaluate if a time dependency occurs.

The time dependency confirms the increasing of the residence time of the drug at the site of action preventing the three-dimensional structure of the gel from being destroyed.

Example 10

Batch 7604

This batch corresponds to the optimized formulation extrapolated by the Full Factorial Design. Batch 7604 aqueous solution was prepared as follows.

| COMPONENTS | %(W/W) |
|---|---|
| Rifamycin SV | 0.500 |
| Poloxamer | 14.900 |
| Sodium Alginate | 0.100 |
| Carboxymethylcellulose Sodium | 0.050 |
| Ascorbic acid | 0.040 |
| Sodium Ascorbate | 0.045 |
| Potassium Sorbate | 0.100 |
| Sodium Benzoate | 0.100 |
| Purified Water | Up to 100 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then, Potassium Sorbate and Sodium Benzoate are added under stirring until complete dissolution is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5°C and 20°C; then, Poloxamer is added. The mixture is kept under stirring until a complete dissolution is achieved and then heated up to room temperature. Then Ethanol 96% is added to the mixture above under stirring until a homogeneous mixture is achieved. Then check the pH and bring it to pH 7.0, if necessary. The mixture is brought to final weight adding Purified water.

The following characteristics have been considered:

1) viscosity of the composition after temperature increase from 25 °C to 37 °C, at 10Hz;
2) as for viscoelastic measurements:

1) Storage elastic modules (G');
2) Loss viscous modules (G");
3) Loss tangent (Tan delta) calculated as ratio between loss module, which is an index of viscous behavior (G") and storage elastic (G') moduli.

The temperature ramp shows a switch between G' and G" modules starting from 29°C up to 31°C. This crossover is associated with an increased elasticity of the sample.

Tan $\delta$ < 1 shows a gel-like behavior meaning a prevalence of the elastic behavior on the viscous one at 37°C. Meanwhile at 25°C tan $\delta$ > 1 shows a liquid-like behaviour confirming the sol-gel transition.

The formulation was subjected to a constant frequency and shear stress for 20 minutes at constant temperature (37°C), to evaluate if a time dependency occurs.

The time dependency confirms the increasing of the residence time of the drug at the site of action preventing the three-dimensional structure of the gel from being destroyed.

Example 11

Batch 7611

Batch 7611 aqueous solution was prepared as follows.

| COMPONENTS | % w/w |
|---|---|
| Rifamycin SV | 1.000 |
| Poloxamer 407 | 16.000 |
| Sodium Alginate | 0.200 |
| Carboxymethylcellulose Sodium | 0.050 |
| Ascorbic Acid | 0.040 |
| Sodium Ascorbate | 0.045 |
| Ethanol | 1.000 |
| Purified Water | Up to 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin is added SV to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

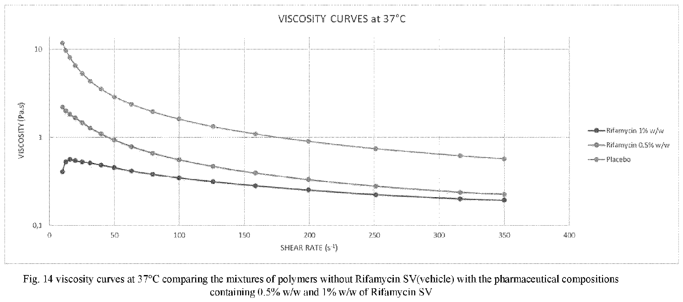

Fig. 14 illustrates the influence of Rifamycin concentration on viscosity behaviour.

Example 12

Batch 7615

Batch 7615 aqueous solution was prepared as follows.

| COMPONENTS | % w/w |
|---|---|
| Rifamycin SV | 0.250 |
| Poloxamer 407 | 15.500 |
| Sodium Alginate | 0.700 |
| Carboxymethylcellulose Sodium | 0.100 |
| Ascorbic Acid | 0.040 |
| Sodium Ascorbate | 0.045 |
| Ethanol | 1.000 |
| Purified Water | Up to 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and broughtto pH 7.0, if necessary. The mixture is brought to final weight adding Purified water.

Example 13

Batch 7616

Batch 7616 aqueous solution was prepared as follows.

| COMPONENTS | % w/w |
|---|---|
| Rifamycin SV | 2.500 |
| Poloxamer 407 | 15.500 |
| Sodium Alginate | 0.300 |
| Carboxymethylcellulose Sodium | 0.100 |
| Ascorbic Acid | 0.040 |
| Sodium Ascorbate | 0.045 |
| Ethanol | 1.000 |
| Purified Water | Up to 100.000 |

In a suitable vessel provided with a stirrer, Purified water (95% of the total amount) is loaded; then, Ascorbic acid and Sodium Ascorbate are added under stirring until complete dissolution is achieved. Then Sodium alginate is added to the mixture above under stirring until a homogeneous mixture is obtained. Then Carboxymethylcellulose Sodium is added to the mixture above under stirring until a homogeneous mixture is achieved. Then the pH of the solution is checked and brought to pH range 6.0-6.5 if needed. Then, Rifamycin SV is added to the mixture above under stirring until complete dissolution is achieved. Then the solution is cooled at a temperature ranging between 5 °C and 20 °C; then, poloxamer is added. The mixture is kept under stirring until a complete dissolution is achieved and then heated up to room temperature. Then Ethanol 96% is added to the mixture above under stirring until a homogeneous mixture is achieved. Then the pH is checked and brought to pH 7.0, if necessary. The mixture is brought to final weight adding Purified water.

Example 14

Rifamycin SV Antibacterial Activity

In patients with ileoanal anastomosis surgery, biopsies obtained from the upstream ileostomy site at the time of stoma closure has shown predominately facultative anaerobes (such as lactobacilli, enterococci and coliforms), a paucity of sulphate-reducing bacteria and low levels of *Clostridium perfringens.* On the other hand, during pouchitis episodes, an increase in the abundance of *Enterobacteriaceae*, reduced abundance of *Bacteroides*, and *Faecalibacterium prausnitzii* has been described.

Rifamycin is a topical antibiotic, and, in fact, it exerts its antibacterial activity against microorganisms that cause gastrointestinal infections, but not systemic infections. Indeed, it is released in the gastrointestinal tract, is excreted, primarily, in feces as unchanged drug.

The Minimum Inhibitory Concentration (MIC) is defined as the lowest concentration expressed in mg/L (equivalent to $\mu$g/mL) of an antimicrobial ingredient or agent that is bacteriostatic (prevents the visible growth of bacteria). MICs are used to evaluate the antimicrobial efficacy of various compounds by measuring the effect of decreasing concentrations of antibiotic over a defined period in terms of inhibition of microbial population growth. MIC is generally regarded as the most basic laboratory measurement of the activity of an antimicrobial agent against an organism. Because a lower MIC value indicates that less of the drug is required in order to inhibit growth of the organism, drugs with lower MIC scores are more effective antimicrobial agents.

Antimicrobial characteristics of Rifamycin sodium salt was assessed on a panel of selected bacteria involved in pouchitis pathogenesis and a standard broth dilution method was used to determine the MIC of Rifamycin. Briefly, two-fold dilutions of the tested compounds were dispensed in 96 round bottom well plates, each dilution was processed in the liquid culture medium suitable for the tested bacterial species. Bacteria inocula were prepared by a suspension of the microbial strain in sterile water saline and adjusted to a concentration of 0.5 McFarland standard corresponding to $1,5 \times 10^8$ CFU/ml. A final bacterial concentration of $0.5\text{-}1 \times 10^5$ CFU/ml was distributed in each well. Each plate was incubated at the appropriate temperature and oxygen concentration for the bacterial species tested (aerobic or anaerobic). Incubation times ranged from a minimum of 24 to a maximum of 72 hours. MIC was recorded when there was visible growth in the positive growth control column (bacterial inoculum without test substance). *In vitro* antimicrobial susceptibility results of the selected bacteria (aerobes and anaerobes) versus tested Rifamycin are shown in Table 3.

TABLE 3

| Rifamycin MIC values tested on selected bacterial strains involved in pouchitis pathogenesis | | |
|---|---|---|
| | **Bacterial Strains** | **Rifamycin** |
| | | **MIC $\mu$g/mL** |
| | *Klebsiella oxytoca* (DSM 6673) | 128 |
| | *K. oxytoca* (DSM 4798) | 256 |
| | *K. oxytoca* (DSM 5175) | 128 |
| Aerobic | *K. oxytoca* (ATCC 700324) | 200 |
| | *K. pneumoniae* (DSM 25721) | 64 |
| | *K. pneumoniae* (DSM 16609) | >64 |
| | | |
| | *Rothia dentocariosa* (DSM 43762) | <0.03 |
| | *Streptococcus pyogenes* (ATCC 700460) | 0.5 |

(continued)

| Rifamycin MIC values tested on selected bacterial strains involved in pouchitis pathogenesis | | |
|---|---|---|
| | *Escherichia coli* (ATCC 35218) | 50 |
| | *E. coli* (ATCC 51446) | 200 |
| | *E. coli* (ATCC 25922) | 64 |
| | *E. coli* (DSM 22312) | 32 |
| | *E. coli* (DSM 22313 ) | 64 |
| | *E. coli (DSM 22664 )* | 32 |
| | *E. coli (ATCC 25922)* | 64 |
| | *E. coli* (ATCC 11775) | 16 |
| | *Enteroccocus faecium (0808711)* | 100 |
| | *E. faecium* (DSM 17050) | 64 |
| | *E. faecium* (DSM 25697) | 1 |
| | *E. faecium* (DSM13590) | 32 |
| | *E. faecium* (09006056) | 100 |
| | *E. faecalis* (DSM 2981) | 200 |
| | *E. faecalis* (DSM 6134) | 50 |
| | | |
| | *E. faecalis* (DSM 2921) | 64 |
| | | |
| | *E. faecalis* (ATCC 29212) | 50 |
| | *E. faecalis* (DSM 2570) | 200 |
| | *E. faecalis* (6172818861) | 12.5 |
| | *E. faecalis* (C 46) | 12.5 |
| | *E. faecalis* (E 83) | 3.12 |
| Anaerobic | *Faecalibacterium prausnitzii* (DSM 107839) | 0.06 |
| | *F. prausnitzii* (DSM 17677) | <0.03 |
| | *F. prausnitzii* (DSM 107840) | <0.03 |
| | *F. prausnitzii* (DSM 107838) | <0.03 |
| | *Bifidobacterium breve* (DSM 20091) | 0.5 |
| | *B. longum* (DSM 20090) | 0.25 |
| | *B. longum* (DSM 20088) | 0.5 |
| | *B. adolescentis* (DSM 20083) | 1 |
| | *B. bifidum* (DSM 20082) | 1 |
| | *Ruminococcus gnavus* (DSM 108212) | <0.03 |
| | *Fusobacterium ulcerans* (DSM 19847) | 16 |
| | *F. nucleatum* (DSM 19507) | 8 |
| | *F. nucleatum* (DSM 15643) | <0.03 |
| | *Eikenella corrodens* (DSM 8340) | <0.3 |
| | *Aggregatibacter actinomycetemcomitans* (DSM 11123) | <0.3 |

(continued)

| Rifamycin MIC values tested on selected bacterial strains involved in pouchitis pathogenesis | | |
|---|---|---|
| | *Porphiromonas gingivalis* (DSM 20709) | <0.3 |
| | *Prevotella intermedia* (DSM 20706) | <0.3 |
| | *Parvimonas micra* (DSM 20468) | <0.3 |
| | *Veillonella parvula* DSM 2007 | <0.03 |
| | *Clostridium difficile* (ATCC 43953) | 0.007 |
| | *C. difficile* (ATCC 17857) | 0.00011 |
| | *C. perfringens* (DSM 11783) | <0.03 |
| | *Bacteroides fragilis* (DSM 2151) | 0.78 |
| | *B. fragilis* (DSM 1396) | *0.39* |
| | *B. fragilis* (DSM 9668) | *100* |
| | *B. fragilis* (DSM 9669) | *100* |
| | *B. fragilis* (DSM 9670) | 50 |
| | *B. fragilis* (DSM 9671) | 50 |

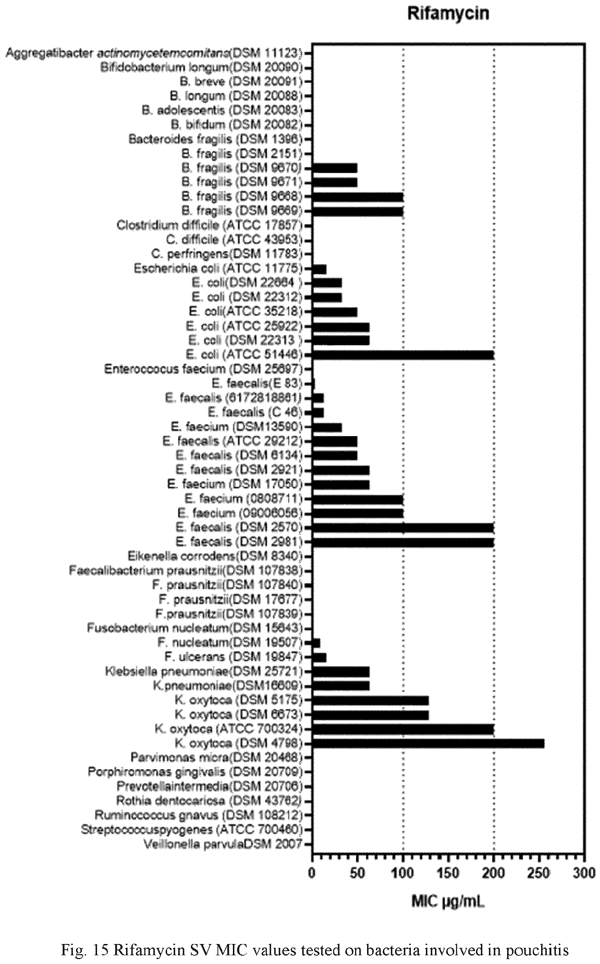

As shown by the results depicted in Table 3 and Fig. 15, Rifamycin shows a powerful antimicrobial activity *in vitro,* with a broad spectrum against Gram-positive and -negative, aerobic and anaerobic bacteria involved in the pathogenesis of several gastrointestinal diseases, including pouchitis.

Example 15

Rifamycin SV Pharmaceutical Composition Antibacterial Activity

Two pharmaceutical compositions were prepared and tested: (1) Rifamycin SV Batch 7570 with its related Placebo Batch 7571 (without Rifamycin SV) and (2) Rifamycin SV Batch 7574 and Placebo Batch 7575 (without Rifamycin SV). The concentration of Rifamycin SV in the pharmaceutical compositions is 5 mg/mL or 7 mM with additional components as listed in Table 3.

TABLE 4

| Composition of Rifamycin and Placebo Pharmaceutical Compositions | | | | |
|---|---|---|---|---|
| **Component** | **Batch 7570 % (W/W)** | **Placebo Batch 7571 % (W/W)** | **Batch 7574 % (W/W)** | **Placebo Batch 7575 % (W/W)** |
| **Rifamycin SV** | 0.5 | 0 | 0.5 | 0 |
| Poloxamer 407 | 16 | 16 | 16 | 16 |
| Sodium Alginate | 0.2 | 0.2 | 0.2 | 0.2 |
| Carboxymethylcellulose Sodium | 0.05 | 0.05 | 0.05 | 0.05 |
| Potassium metabisulfite | 0.012 | 0.012 | 0 | 0 |
| Ascorbic acid | 0.043 | 0.043 | 0.04 | 0.04 |
| Sodium Ascorbate | 0.048 | 0.048 | 0.045 | 0.045 |
| Ethanol | 0 | 0 | 1 | 1 |
| Purified Water | Up to 100 | Up to 100 | Up to 100 | Up to 100 |

Antibacterial activities for Rifamycin SV formulations, Batch 7570 and Batch 7574 and their related placebo formulations (7571 and 7575) were determined by agar diffusion assay in solid inoculated culture media. Agar well diffusion method is widely used to evaluate antibiotic activity and, in this case, allow for the combined testing of antibacterial activity together with formulation diffusion capability.

Briefly, agar medium was prepared according to the manufacturer's instructions and autoclaved at 121 °C for 15 minutes. Subsequently, the temperature was lowered slightly above the agarization point by a thermostatically controlled bath and then was added to media live bacterial inocula at a final concentration of $1\times10^5$ CFU/ml. The inoculated media were dispensed into Petri dishes and allowed to solidify. Holes (6 mm diameter) were then made for each petri dish to dispense the test substance (40μl). Finally, plates were incubated at the appropriate temperature and oxygen concentration for the bacterial species tested (aerobic or anaerobic) to allow the bacterial growth and the compound diffusion through the agar plate. Incubation times ranged from a minimum of 24 to a maximum of 72 hours. Antimicrobial susceptibility was assessed as the extent of the halo of inhibition (mm) exerted by the tested compound in counteracting microbial growth. Experiments were conducted in duplicate, and results expressed as mean and standard deviation. Gel formulation and related placebo were tested in parallel. *In vitro* antimicrobial susceptibility results, determined by agar diffusion on the selected bacteria (aerobes and anaerobes) versus tested formulations are shown in Table 5, larger halo inhibition diameter corresponds to higher anti-bacterial activity.

TABLE 5

Inhibition halos (mm) for Rifamycin SV gel formulation 7570, placebo formulation 7571,
Rifamycin sv gel formulation 7574 and placebo formulation 7575

|  | Zone of inhibition | Rifamycin formulation 7570 Ø mm | | Placebo formulation 7571 Ø mm | | Rifamycin formulation 7574 Ø mm | | Placebo formulation 7575 Ø mm | |
|---|---|---|---|---|---|---|---|---|---|
|  |  | Mean | SD | Mean | SD | Mean | SD | Mean | SD |
| *Aerobic* | *Klebsiella oxytoca* (DSM 6673) | 0 | 0 | 0 | 0 | 0 | 0 | 0 | 0 |
|  | *K. oxytoca* (DSM 4798) | 0 | 0 | 0 | 0 | 0 | 0 | 0 | 0 |
|  | *K. oxytoca* (DSM 5175) | 0 | 0 | 0 | 0 | 0 | 0 | 0 | 0 |
|  | *Rothia dentocariosa* (DSM 43762) | 39.5 | 0.71 | 0 | 0 | 38.5 | 0.71 | 0 | 0 |
|  | *Streptococcus pyogenes* (ATCC 700460) | 42 | 0 | 0 | 0 | 42 | | 0 | 0 |
|  | *Enterococcus faecalis* (E 83) | 24.5 | 0.71 | 0 | 0 | 22.5 | 0.71 | 0 | 0 |
|  | *E. faecium* (DSM 25697) | 20 | 0 | 0 | 0 | 22.5 | 0.71 | 0 | 0 |
| *Anaerobic* | *Faecalibacterium prausnitzii* (DSM 107839) | 42 | 0 | 0 | 0 | 44 | 0 | 0 | 0 |
|  | *F. prausnitzii* (DSM 17677) | 40 | 0 | 0 | 0 | 44 | 0 | 0 | 0 |
|  | *F. prausnitzii* (DSM 107840) | 35.5 | 0.71 | 0 | 0 | 36.5 | 0.71 | 0 | 0 |
|  | *F. prausnitzii* (DSM 107838) | 60 | 0 | 0 | 0 | 60 | 0 | 0 | 0 |
|  | *Bifidobacterium breve* (DSM 20091) | 41.5 | 0.71 | 0 | 0 | 42.5 | 2.12 | 0 | 0 |
|  | *B. longum* (DSM 20090) | 33 | 4.24 | 0 | 0 | 31 | 1.41 | 0 | 0 |
|  | *B. longum* (DSM 20088) | 68 | 0 | 0 | 0 | 70 | 0 | 0 | 0 |
|  | *B. adolescentis* (DSM 20083) | 46 | 2.83 | 0 | 0 | 31 | 1.41 | 0 | 0 |
|  | *B. bifidum* (DSM 20082) | 36 | 0 | 0 | 0 | 37 | 1.41 | 0 | 0 |
|  | *Clotridium perfringens* (DSM 11783) | 31 | 1.41 | 0 | 0 | 30 | 0 | 0 | 0 |
|  | *Ruminococcus gnavus* (DSM 108212) | 32 | 0 | 0 | 0 | 30 | 0 | 0 | 0 |
|  | *Fusobacterium ulcerans* (DSM 19847) | 18 | 0 | 0 | 0 | 20 | 0 | 0 | 0 |
|  | *F. nucleatum* (DSM 19507) | 49 | 1.41 | 0 | 0 | 49.5 | 0.41 | 0 | 0 |
|  | *F. nucleatum* (DSM 15643) | 60 | 0 | 0 | 0 | 60 | 0 | 0 | 0 |

| *Eikenella corrodens* (DSM 8340) | 48 | 0 | 0 | 0 | 50 | 0 | 0 | 0 |
|---|---|---|---|---|---|---|---|---|
| *Aggregatibacter actinomycetemcomitans* (DSM 11123) | 46 | 0 | 0 | 0 | 46 | 0 | 0 | 0 |
| *Porphiromonas gingivalis* (DSM 20709) | 27.5 | 0.71 | 0 | 0 | 28 | 0 | 0 | 0 |
| *Prevotella intermedia* (DSM 20706) | 51 | 1.41 | 0 | 0 | 53 | 1.41 | 0 | 0 |
| *Parvimonas micra* (DSM 20468) | 59 | 1.41 | 0 | 0 | 56 | 0 | 0 | 0 |
| *Veilonella parvula* (DSM 2007) | 22 | 0 | 0 | 0 | 21.5 | 0.71 | 0 | 0 |

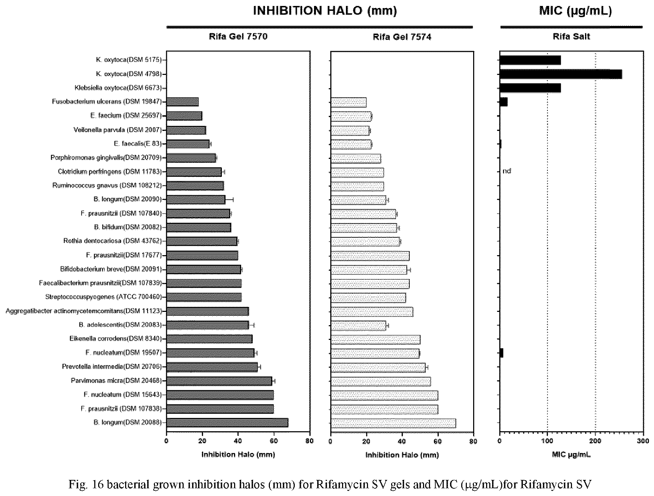

The two batches tested (7570 and 7574) showed a remarkable growth inhibition of bacteria involved in pouchitis pathogenesis. As shown in Fig. 16, results confirmed a correspondence between the MIC values obtained for Rifamycin as salt only (reported in Table 3) and the inhibition halos of Rifamycin SV formulation batches 7570 and 7574 which gel; indeed, for the same bacterial strains, lower MIC values correspond to inhibition halos with larger diameter. Additionally, the two placebo formulations (7571 and 7575) did not exert any antimicrobial activity against the tested microbial strains confirming specific Rifamycin SV formulation antibacterial activity.

Example 16

Rifamycin SV Pharmaceutical Composition Anti Inflammatory Activities via PXR activation

Anti-inflammatory activities for Rifamycin SV formulations, Batch 7570 and Batch 7574 and their related placebo formulations (7571 and 7575) were determined using *in vitro* assays to measure activation of the Pregnane X Receptor (PXR) transcription factor, inhibition of Nuclear Factor kB (NFkB) transcription factor and inhibition of the mechanistic target of rapamycin (mTOR) pathway.

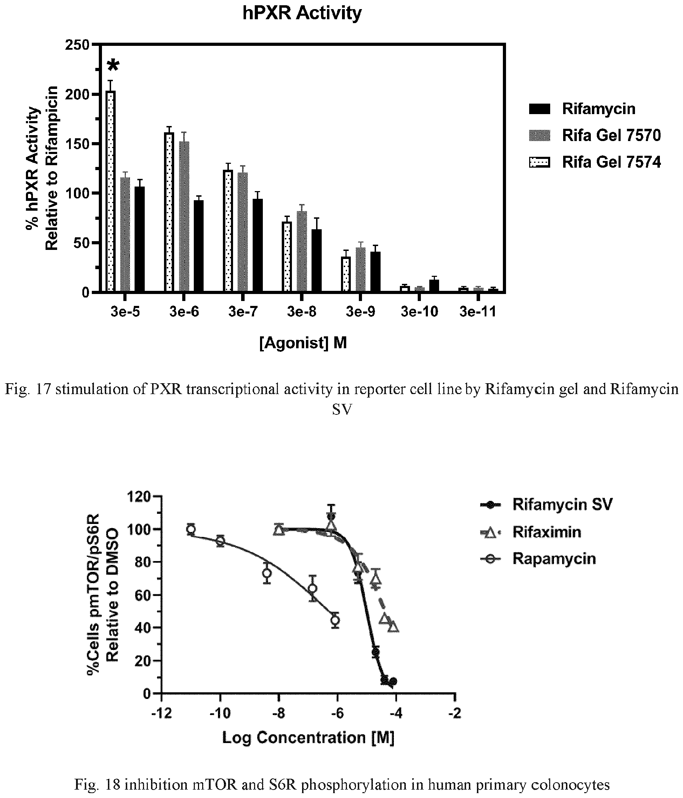

In addition to its role in detoxification mechanisms, the transcription factor PXR has been shown to indirectly inhibit inflammation through repression of another transcription factor, NFkB. This ultimately leads to lower expression of multiple inflammatory cytokines including IL8 in intestinal cells [Rosette et al., 2019]. A Human embryonic kidney (HEK293t) reporter cell line expressing a hybrid PXR protein was used to compare the anti-inflammatory activity of the Rifamycin formulations with the Rifamycin sodium salt. Cells were seeded onto 96 well collagen coated plates and treated for 24h with the test solutions in a 7-point log dose titration. Luminescence proportional to PXR activity was quantified using a luminometer. As shown in Figure 17, both formulations containing Rifamycin SV showed enhanced stimulatory efficacy on PXR transcriptional activity relative to Rifamycin when tested at the concentration of $3\times10^{-6}$ M. The maximum activity by Rifampicin was used as the comparator (100% stimulation) because this is the reference standard for PXR activation in this reporter assay system. The concentration of $3\times10^{-6}$ M is readily achieved in the proposed enema gel formulation for pouchitis, which contains 7 mM, or 200-fold more concentrated than the highest tested in the PXR assay. In addition, the highest anti-bacterial MIC value of 200 $\mu$g/mL (0.3 mM) is 23-fold lower than the proposed 7 mM pouchitis formulation. Therefore, the required concentrations for both anti-inflammatory (PXR activity) and anti-bacterial (MIC) are well below the 7 mM Rifamycin concentration in the gel formulation. Both placebo gel formulations (also referred to as vehicles) and DMSO alone did not induce PXR transcriptional activity attesting specificity of the formulation activity (data not shown).

Example 17

Rifamycin SV Anti-inflammatory Activity via Inhibition of the mTOR Pathway

The mechanistic target of rapamycin (mTOR) is a serine/threonine kinase that exists as the catalytic subunit of two biochemically distinct complexes called mTOR complex 1 (mTORC1) and mTORC2. Classically, mTORC1 controls cell growth in response to nutrient availability and growth factors. However, recent evidence revealed the involvement of immune cell-specific mTORC1 in various inflammatory diseases [Lin X (2018)]. The mTOR pathway promotes expression of key effector and regulatory cytokines that are implicated in UC, such as IL10, IFN$\gamma$, and IL17 [Xie et al (2020)] demonstrated an essential role of epithelial mTORC1 in UC pathogenesis and established a communication link between colonic epithelium and immune cells during UC development. This study used two genetic mouse models and importantly, biopsies from UC patients. mTOR inhibitors such as rapamycin have anti-inflammatory effects in models of experimental colitis and the antibiotic rifaximin has been shown to inhibit the mTOR pathway *in vitro* in colonic epithelial cells.

The inflamed J pouch has an inflammatory signature similar to the colon of UC patients, indicating that they are driven by similar inflammatory mechanisms and may therefore respond to similar therapeutics. Thus, mTOR inhibitors may also be effective in treating pouchitis.

To evaluate Rifamycin activity on the mTOR pathway, primary colonic epithelial cells were serum starved for 24 hr and restimulated with serum for 48 hr to induce phosphorylation of mTOR and its downstream target protein S6R. Serum stimulated cells were treated with Rifamycin SV, Rifaximin, the positive control Rapamycin, or the negative vehicle control DMSO. Cells were fixed, stained with antibodies against pmTOR and pS6R then analyzed by flow cytometry. The percentage of cells that stain positively for both antibodies were quantified and normalized to the DMSO control (set to 100%).

Fig. 18 shows the % cells that stained positive for anti-pmTOR and anti-pS6R relative to DMSO. The rank order of mTOR pathway potency is Rapamycin > Rifamycin SV > Rifaximin (IC$_{50}$ $5.3 \times 10^{-7}$, $9.9 \times 10^{-6}$, $4.3 \times 10^{-5}$, respectively). Thus, Rifamycin SV is 4-fold more potent than Rifaximin and only 20-fold less potent than the standard reference mTOR pathway inhibitor, Rapamycin.

**BIBLIOGRAPHY**

Akiyama, S., et al. (2021). Pouchitis in inflammatory bowel disease: a review of diagnosis, prognosis, and treatment. Intest Res 19(1):1-11. doi:10.5217/ir.2020.00047

Al-Joufi, F., et al. (2021). Mucoadhesive In Situ Rectal Gel Loaded with Rifampicin: Strategy to Improve Bioavailability and Alleviate Liver Toxicity. Pharmaceutics, 13(3), 336. doi: 10.3390/pharmaceutics13030336. PMID: 33807729; PMCID: PMC8001001.

Brereton, R.G., (2003). Chemometrics: data analysis for the laboratory and chemical plant. John Wiley & Sons Ltd, Chichester.

Devlin, J.C., et al. (2020). Transcriptional atlas of ileal-anal pouch immune cells from ulcerative colitis patients. bioRxiv preprint; doi: https://doi.org/10.1101/2020.07.31.231308.

Draper, N.R. and Smith, H. (1981), Applied regression analysis, New York: Wiley & sons, 412-419.

Huang, y., et al. (2017). Early transcriptomic changes in the ileal pouch provide insight into the molecular pathogenesis of pouchitis and ulcerative colitis. Inflamm Bowel Dis 23:366.

Isaacs, K., et al. (2007). Rifaximin for the treatment of active pouchitis: a randomized, double-blind, placebo-controlled pilot study. Inflamm Bowel Dis, 2007; 13: 1250-1255.

Li, Y. and Shen, B. (2014). Management of acute and chronic pouchitis. Medical Therapy of Ulcerative Colitis, Springer, New York, pp. 367-376.

McLaughlin, S.D., et al. (2010). The bacteriology of pouchitis: a molecular phylogenetic analysis using 16S rRNA gene cloning and sequencing. Ann Surg 252: 90-8.

McLaughlin, S.D. and Clark, S.K. (2010). The bacterial pathogenesis and treatment of pouchitis. Therapeutic advances in Gastroenterology, 2010; 3 (6): 335-348.

Reshef, L., et al. (2015). Pouch inflammation is associated with a decrease in specific bacterial taxa. Gastroenterology 149: 718-27.

Rifamycin Investigator's Drug Brochure, Version 7 (Release date: 28 September 2020).

Rosette, C. et al. (2013). Anti-inflammatory and immunomodulatory activities of rifamycin SV. Int J Antimicrob Agents 42:182-186.

Rosette, C., et al. (2019). Rifamycin SV exhibits strong anti-inflammatory in vitro activity through pregnane X receptor stimulation and NFkB inhibition. Drug Metabolism and Pharmacokinetics, 34(3): 172-180.

Sandborn, W.J., et al. (1994). Pouchitis after ileal pouch-anal anastomosis: a Pouchitis Disease Activity Index. Mayo Clin Proc 69(5):409-15.

Schieffer, K.M., et al. (2016). Review article: the pathogenesis of pouchitis. Aliment Pharmacol Ther 2016, 44:817-835.

Shen, B. and Lashner, B.A. (2008). Diagnosis and treatment of pouchitis. Gastroenterol Hepatol; 4 (5): 355-361.

Smith, F.M., et al. (2005). A characterization of anaerobic colonization and associated mucosal adaptations in the undiseased ileal pouch. Colorectal Dis 7: 563-70.

Tyler, A.D., et al. (2013). Characterization of the gut associated microbiome in inflammatory pouch complications following ileal pouch-anal anastomosis. PLoS ONE 2013; 8: e66934.

Xie, Y., et al. (2020). Gut epithelial TSC1/mTOR controls RIPK3-dependent necroptosis in intestinal inflammation and cancer. J Clin Invest 130:2111.

**Claims**

1. A pharmaceutical composition comprising:

   a therapeutically effective amount of rifamycin SV; and
   a polymer mixture;
   wherein the pharmaceutical composition is a liquid at room temperature and is capable of forming a gel in situ at body temperature.

2. The pharmaceutical composition of claim 1, wherein the polymer mixture comprises:

   at least one thermo-responsive polymer;
   at least one ion-sensitive polymer; and
   at least one bio-adhesive polymer;

3. The pharmaceutical composition of claim 1 or 2, wherein the pharmaceutical composition is an enema.

4. The pharmaceutical composition of claim 2 or 3, wherein the thermo-responsive polymer is selected from the group consisting of: polyoxyethylene-polyoxypropylene block copolymers, poly(ethylene glycol)/poly(lactide-coglicolide) block copolymers (PEG-PLGA), poly(ethylene glycol)-poly(lactic acid)-poly(ethylene glycol) (PEG-PLA-PEG), poly(N-isopropylacrylamide), and cellulose derivatives.

5. The pharmaceutical composition of claim 2, 3 or 4, wherein the thermo-responsive polymer is a polyoxyethylene-polyoxypropylene block copolymer selected from the group consisting of: poloxamer 124, poloxamer 188, poloxamer 237, poloxamer 338, and poloxamer 407.

6. The pharmaceutical composition of any of claims 2 to 4, wherein the thermo-responsive polymer is a cellulose derivative selected from the group consisting of methylcellulose (MC), hydroxypropylmethylcellulose (HPMC) and mixtures thereof.

7. The pharmaceutical composition of any of claims 2 to 5, wherein the thermo-responsive polymer is polyoxamer 188, poloxamer 407, or mixtures thereof.

8. The pharmaceutical composition of any one of claims 2 to 7, wherein the ion-sensitive polymer is a polysaccharide.

9. The pharmaceutical composition of any of claims 2 to 8, wherein the ion-sensitive polymer is selected from the group consisting of: carrageenan, gellan gum, pectin, alginic acid, salts thereof, and mixtures thereof.

**10.** The pharmaceutical composition of any of claims 2 to 9, wherein the ion-sensitive polymer is sodium alginate.

**11.** The pharmaceutical composition of any of claims 2 to 10, wherein the bio-adhesive polymer is selected from the group consisting of: chitosan, hyaluronic acid and salts thereof, cellulose derivatives, polyvinyl alcohol, polyvinyl pyrrolidone, polyacrylic acid, polyethylene oxides, tragacanth, sodium alginate, xanthan gum, gelatin, pectin, and mixtures thereof.

**12.** The pharmaceutical composition of any of claims 2 to 11, wherein the bio-adhesive polymer is a cellulose derivative selected from the group consisting of: methyl cellulose, hydroxy propyl methylcellulose, hydroxy propyl cellulose, carboxymethyl cellulose sodium and mixtures thereof.

**13.** The pharmaceutical composition of any of claims 2 to 12, wherein the bio-adhesive polymer is a sodium salt of carboxymethyl cellulose.

**14.** The pharmaceutical composition of any of the preceding claims, wherein the pharmaceutical composition further comprises at least one pharmaceutically acceptable excipient selected from the group consisting of: an anti-oxidant, a preservative, and a combination thereof.

**15.** A pharmaceutical composition comprising a therapeutically effective amount of rifamycin SV and a polymer mixture for use in treating pouchitis;
wherein the pharmaceutical composition is administered rectally, is a liquid at room temperature and forms a gel in situ at a body temperature of said patient.

**16.** The pharmaceutical composition for use as claimed in claim 15, wherein said pharmaceutical composition is an enema.

**17.** The pharmaceutical composition for use as claimed in claim 15 or 16 to alleviate and/or reduce one or more clinical symptoms selected from the group consisting of: increased frequency of evacuations, reduction in fecal consistency, rectorrhagia, pain, cramp-like abdominals, urgency, tenesmus, fecal incontinence, fever and extraintestinal manifestations.

**18.** The pharmaceutical composition for use as claimed in claim 15 or 16 to alleviate and/or reduce the occurrence of one or more endoscopy symptoms selected from the group consisting of: mucosal edema, granularity, contact bleeding, loss of vascular pattern, hemorrhage, and ulceration.

**19.** The pharmaceutical composition for use as claimed in any of claims 15 to 18 wherein said pharmaceutical composition is administered daily or twice a day.

**20.** The pharmaceutical composition for use as claimed in any of claims 14 to 19 wherein said rectal administration occurs for at least two weeks.

Fig. 1 Simplex Centroid Design

Fig. 2 viscosity curves of Runs from 1 to 6 and from 8 to 9 with Rifamycin SV both at 25°C and 37°C

Fig. 3 temperature ramp for Run 2 at 10 Hz

Fig. 4 Fmax values for Runs 1-10 in presence and absence (blank) of mucine at 8% w/w

Fig. 5 Work of Adhesion for Runs 1-10 in presence and absence (blank) of mucine at 8% w/w

Fig. 6 contour plot of normalized Work of Adhesion

Fig. 7 Full Factoria Design

Fig. 8 logarithmic viscosity curves of Runs 1-14 of Full Factorial Design with Rifamycin

Fig. 9 Temperature ramp of vehicle 9

Fig. 10 Temperature ramp of vehicle 14

### Contour Plot of Δwork/ work vs CMC; Alginate

Fig. 11 contour plot of normalized Work of Adhesion

Fig. 12 Loss tangent (Tan δ) as function of frequency at 25°C and 40°C

Fig. 13 Time dependency profiles of both Placebo and Rifamycin SV formulations

Fig. 14 viscosity curves at 37°C comparing the mixtures of polymers without Rifamycin SV(vehicle) with the pharmaceutical compositions containing 0.5% w/w and 1% w/w of Rifamycin SV

Fig. 15 Rifamycin SV MIC values tested on bacteria involved in pouchitis

Fig. 16 bacterial grown inhibition halos (mm) for Rifamycin SV gels and MIC (μg/mL)for Rifamycin SV

Fig. 17 stimulation of PXR transcriptional activity in reporter cell line by Rifamycin gel and Rifamycin SV

Fig. 18 inhibition mTOR and S6R phosphorylation in human primary colonocytes

Europäisches
Patentamt

European
Patent Office

Office européen
des brevets

# EUROPEAN SEARCH REPORT

**Application Number**

EP 21 20 3010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 3 501 496 A1 (COSMO TECHNOLOGIES LTD [IE]) 26 June 2019 (2019-06-26) | 1-14 | INV.<br>A61K9/08 |
| A | * paragraph [0203]; claims 1,12; example 11 * | 15-20 | A61P1/00<br>A61K47/10<br>A61K47/36 |
| A | WO 2009/108814 A1 (SALIX PHARMACEUTICALS LTD [US]; FORBES WILLIAM [US] ET AL.) 3 September 2009 (2009-09-03) * claims 1,36; example 1 * | 1-20 | A61K47/38 |
| A | Kumbhar Amruta B ET AL: "In situ gel forming injectable drug delivery system", International journal of pharmaceutical sciences and research, 1 February 2013 (2013-02-01), page 597, XP055903025, Retrieved from the Internet: URL:https://ijpsr.com/bft-article/in-situ-gel-forming-injectable-drug-delivery-system/#:~:text=In%20situ%20gel%20forming%20injectable%20drug%20delivery%20system%20is%20the,and%20localized%2C%20prolonged%20drug%20delivery. [retrieved on 2022-03-18] * abstract * | 1-20 | |

TECHNICAL FIELDS
SEARCHED (IPC)

A61K

The present search report has been drawn up for all claims

| Place of search | Date of completion of the search | Examiner |
|---|---|---|
| The Hague | 18 March 2022 | Konter, Jörg |

## EP 4 162 928 A1

### ANNEX TO THE EUROPEAN SEARCH REPORT
### ON EUROPEAN PATENT APPLICATION NO.

EP 21 20 3010

This annex lists the patent family members relating to the patent documents cited in  the above-mentioned European search report.
The members are as contained in the European Patent Office EDP file on
The European Patent Office is in no way liable for these particulars which are merely given for the purpose of information.

18-03-2022

| Patent document cited in search report | | Publication date | Patent family member(s) | | Publication date |
|---|---|---|---|---|---|
| EP 3501496 | A1 | 26-06-2019 | BR 112020012519 | A2 | 23-02-2021 |
| | | | CA 3081633 | A1 | 27-06-2019 |
| | | | CN 111511352 | A | 07-08-2020 |
| | | | EP 3501496 | A1 | 26-06-2019 |
| | | | EP 3727453 | A1 | 28-10-2020 |
| | | | JP 2021506913 | A | 22-02-2021 |
| | | | KR 20200103012 | A | 01-09-2020 |
| | | | RU 2020124103 | A | 24-01-2022 |
| | | | US 2021154300 | A1 | 27-05-2021 |
| | | | WO 2019122253 | A1 | 27-06-2019 |
| WO 2009108814 | A1 | 03-09-2009 | AU 2009219240 | A1 | 03-09-2009 |
| | | | BR PI0908026 | A2 | 21-07-2015 |
| | | | CA 2716578 | A1 | 03-09-2009 |
| | | | CN 101959412 | A | 26-01-2011 |
| | | | DK 2252148 | T3 | 06-05-2019 |
| | | | EP 2252148 | A1 | 24-11-2010 |
| | | | EP 3563850 | A1 | 06-11-2019 |
| | | | ES 2727728 | T3 | 18-10-2019 |
| | | | KR 20100122937 | A | 23-11-2010 |
| | | | NZ 587098 | A | 28-09-2012 |
| | | | PL 2252148 | T3 | 30-09-2019 |
| | | | PT 2252148 | T | 23-01-2020 |
| | | | RU 2010139475 | A | 10-04-2012 |
| | | | RU 2014104362 | A | 20-08-2015 |
| | | | US 2011118295 | A1 | 19-05-2011 |
| | | | US 2013137713 | A1 | 30-05-2013 |
| | | | US 2016000765 | A1 | 07-01-2016 |
| | | | WO 2009108814 | A1 | 03-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102151242
- WO 2019122253 A
- WO 2019122593 A

**Non-patent literature cited in the description**

- *Clin Drug Investig,* July 2019, vol. 39 (7), 691-697
- *CHEMICAL ABSTRACTS,* 14897-39-3
- **AKIYAMA, S. et al.** Pouchitis in inflammatory bowel disease: a review of diagnosis, prognosis, and treatment. *Intest Res,* 2021, vol. 19 (1), 1-11
- **AL-JOUFI, F. et al.** Mucoadhesive In Situ Rectal Gel Loaded with Rifampicin: Strategy to Improve Bioavailability and Alleviate Liver Toxicity. *Pharmaceutics,* 2021, vol. 13 (3), 336
- **BRERETON, R.G.** Chemometrics: data analysis for the laboratory and chemical plant. John Wiley & Sons Ltd, 2003
- **DEVLIN, J.C. et al.** Transcriptional atlas of ileal-anal pouch immune cells from ulcerative colitis patients. *bioRxiv preprint,* 2020
- **DRAPER, N.R. ; SMITH, H.** Applied regression analysis. Wiley & sons, 1981, 412-419
- **HUANG, Y. et al.** Early transcriptomic changes in the ileal pouch provide insight into the molecular pathogenesis of pouchitis and ulcerative colitis. *Inflamm Bowel Dis,* 2017, vol. 23, 366
- **ISAACS, K. et al.** Rifaximin for the treatment of active pouchitis: a randomized, double-blind, placebo-controlled pilot study. *Inflamm Bowel Dis,* 2007, vol. 13, 1250-1255
- Management of acute and chronic pouchitis. **LI, Y ; SHEN, B.** Medical Therapy of Ulcerative Colitis. Springer, 2014, 367-376
- The bacteriology of pouchitis: a molecular phylogenetic analysis using 16S rRNA gene cloning and sequencing. **MCLAUGHLIN, S.D. et al.** Ann Surg. 2010, vol. 252, 90-8
- **MCLAUGHLIN, S.D. ; CLARK, S.K.** The bacterial pathogenesis and treatment of pouchitis. *Therapeutic advances in Gastroenterology,* 2010, vol. 3 (6), 335-348

- **RESHEF, L. et al.** Pouch inflammation is associated with a decrease in specific bacterial taxa. *Gastroenterology,* 2015, vol. 149, 718-27
- **ROSETTE, C. et al.** Anti-inflammatory and immunomodulatory activities of rifamycin SV. *Int J Antimicrob Agents,* 2013, vol. 42, 182-186
- **ROSETTE, C. et al.** Rifamycin SV exhibits strong anti-inflammatory in vitro activity through pregnane X receptor stimulation and NFkB inhibition. *Drug Metabolism and Pharmacokinetics,* 2019, vol. 34 (3), 172-180
- **SANDBORN, W.J. et al.** Pouchitis after ileal pouch-anal anastomosis: a Pouchitis Disease Activity Index. *Mayo Clin Proc,* 1994, vol. 69 (5), 409-15
- **SCHIEFFER, K.M. et al.** Review article: the pathogenesis of pouchitis. *Aliment Pharmacol Ther,* 2016, vol. 44, 817-835
- **SHEN, B. ; LASHNER, B.A.** Diagnosis and treatment of pouchitis. *Gastroenterol Hepatol,* 2008, vol. 4 (5), 355-361
- **SMITH, F.M. et al.** A characterization of anaerobic colonization and associated mucosal adaptations in the undiseased ileal pouch. *Colorectal Dis,* 2005, vol. 7, 563-70
- **TYLER, A.D. et al.** Characterization of the gut associated microbiome in inflammatory pouch complications following ileal pouch-anal anastomosis. *PLoS ONE,* 2013, vol. 8, e66934
- **XIE, Y. et al.** Gut epithelial TSC1/mTOR controls RIPK3-dependent necroptosis in intestinal inflammation and cancer. *J Clin Invest,* 2020, vol. 130, 2111